# EUROPÄISCHE PATENTANMELDUNG

(11) **EP 3 750 936 A1**
(43) Veröffentlichungstag der Anmeldung: **16.12.2020**
(21) Anmeldenummer: 19179686.1
(22) Anmeldetag: 12.06.2019
(51) Int. Cl.: C08G 63/08, C08G 63/82, C07D 315/00, C07D 305/12, B01J 31/02, B01J 31/18, B01J 31/20, B01J 31/22

(54) **VERFAHREN ZUR CARBONYLIERUNG VON EPOXIDEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Gegenstand der Erfindung ist ein Verfahren zur Carbonylierung von Epoxiden in Gegenwart von Katalysator-Systemen, wobei das Katalysatorsystem eine Molybdän-basierte Verbindung enthält. Ein weiterer Erfindungsgegenstand sind Carbonylierungsprodukte sowie Carbonylierungsfolgeprodukte und die Verwendung erfindungsgemäßer Katalysator-Systeme zur Carbonylierung von Epoxiden.

## Beschreibung

Gegenstand der Erfindung ist ein Verfahren zur Carbonylierung von Epoxiden in Gegenwart von Katalysator-Systemen, wobei das Katalysatorsystem eine Molybdän-basierte Verbindung enthält. Ein weiterer Erfindungsgegenstand sind Carbonylierungsprodukte sowie Carbonylierungsfolgeprodukte und die Verwendung erfindungsgemäßer Katalysator-Systeme zur Carbonylierung von Epoxiden.

In DE 10235316 A1 wird ein Verfahren zur Herstellung von Lactonen durch katalytische Carbonylierung von Oxiranen offenbart, wobei ein Katalysatorsystem aus a) mindestens einem Carbonylierungskatalysator A aus neutralen oder anionischen Übergangsmetallkomplexen von Metallen der Gruppen 5 bis 11 des Periodensystems der Elemente und b) mindestens einer chiralen Lewis-Säure B als Katalysator eingesetzt wird, mit Ausnahme von [(salph)Al(THF)2][Co(CO)4]. In WO 2006/058681 A2 wird ein Verfahren zur Herstellung von enantiomerenangereicherten Lactonen durch katalytische Carbonylierung von Lactonen zu Anhydriden in Anwesenheit eines neutralen oder anionischen Übergangsmetall-Komplexen beschrieben.

In WO 2016/015019 A1 wird eine Methode zur Darstellung von Aluminium-Komplexen, mit dem Nutzen die Carbonylierung von Epoxiden zu katalysieren, beschrieben.

In JP2013173090 wird die Carbonylierung von Epoxiden unter Verwendung immobilisierter Katalysatorsysteme beschrieben.

In EP 0176370 A1 wird einen Methode zur Darstellung von Lactonen über die cyclisierende Veresterung von ungesättigten Alkoholen mit Kohlenmonoxid unter Anwesenheit eines Katalysators beschrieben. Hier wird Mo als Bestandteil eines bi-metallischen Katalysatorsystems beschrieben. *beta*-Lactone, d.h. cyclische 4-Ring-Ester, werden weder beschrieben noch beansprucht. Auch Epoxiden als mögliche Substrate und/oder Intermediate werden nicht beschrieben und nicht beansprucht.

In WO 2011/163309 A2 wird ein zweistufiges Verfahren zur Herstellung von Polyhydroxybutyrat- und/oder Polyhydroxypropionat-Homopolymeren mit anschließender thermischer Zersetzung zu ungesättigten Säuren wie Crotonsäure und/oder Acrylsäure beschrieben. In der ersten Stufe werden durch die Carbonylierung von Epoxiden die entsprechenden *beta*-Lactone hergestellt und in der zweiten Stufe zu den entsprechenden Homopolymeren umgesetzt.

Cobalt- und Molybdänverbindungen unterscheiden sich erheblich in ihrer regulatorischen Einstufung. Die US-Bundesbehörde OSHA schreibt einen Zeitgewichteten Durchschnittswert für Arbeitsplatzgrenzwerte von ≤ 0,1 ppm für Cobaltcarbonyle und Cobalthydrocarbonyle vor (vgl. Clinical Toxicology, 1999, 37, 201-216), wohingegen ein Zeitgewichteten Durchschnittswert für Expositionsgrenzwerte für lösliche Molybdänverbindungen bei 5 ppm und für unlösliche Molybdänverbindungen bei 10 ppm liegt (vgl. Clinical Toxicology, 1999, 37, 231-237).

Die Weltproduktion an Cobalt lang 2016 bei 123.000 Tonnen (laut Department of Natural Resources, Canada). Wohingegen die Weltproduktion an Molybdän 2007 bei ca. 211.000 Tonnen lag (vgl. Statistik der United States Geological Society). Im Laufe des Jahres 2018 wurde der Preis mit 25 $/kg am Rohstoffmarkt angegeben (siehe London Metal Exchange, historische Preisdaten für Molybdän).

Ausgehend vom Stand der Technik war es Aufgabe der vorliegenden Erfindung die Nachteile von Cobalt-basierten Katalysatoren für die Carbonylierung von Epoxiden abzuhelfen.

Es war daher Aufgabe der vorliegenden Erfindung, ein vereinfachtes Verfahren für die Carbonylierung von Epoxiden zu Carbonylierungsprodukten unter Einsatz eines Katalysatorsystems bereitzustellen, wobei im Speziellen *beta*-Lactone, cyclische Esteretherverbindungen und als deren Folgeprodukte Polyhydroxyalkanoat-(Co)polymere, insbesondere Polyhydroxybutyrat- und/oder Polyhydroxypropionat-(Co)polymeren, durch eines direktes und einstufiges Verfahren bereitgestellt werden sollen.

Hierbei soll ein chemisch stabileres Katalysatorsystem verwendet werden, so dass eine längere Lagerung und/oder eine technisch einfachere Lagerung, Handling und Nutzung gegenüber aus dem Stand der Technik vorbeschriebenen Cobalt-basierten Systemen möglich ist. In den im Stand der Technik vorbeschriebenen Cobalt-basierten Systemen liegt Cobalt in der sehr empfindlichen Oxidationsstufe -1 vor und kann äußerst leicht mit Oxidanzien aller Art sowie Hydroxyverbindungen oder Wasser degradiert werden. Daneben sollen sich die erfindungsgemäßen Katalysatorsysteme durch eine bessere technische Verfügbarkeit und eine geringere Toxizität gegenüber den Cobalt-basierten Systemen auszeichnen, wobei auch eine direkte Weiterverarbeitung der Carbonylierungsprdukte zu Carbonylierungsfolgeprodukten, beispielsweise der Umsetzung zu Polyurethanen ohne vorherige Abtrennung des Katalysatorsystems möglich ist.

Überraschend wurde gefunden, dass die erfindungsgemäße Aufgabe gelöst wird durch ein Verfahren zur Carbonylierung von Epoxiden in Gegenwart von Katalysator-Systemen, dadurch gekennzeichnet, dass das Katalysatorsystem eine Molybdän-basierte Verbindung enthält.

In einer Ausführungsform des erfindungsgemäßen Verfahren wird die Molybdän-basierte Verbindung in Mengen von 0,0001 mol-% bis 20 mol-% bezogen auf die Stoffmenge an Epoxid verwendet.

In einer Ausführungsform des erfindungsgemäßen Verfahrens weist die Molybdän-basierte Verbindung eine Oxidationsstufe von null auf.

In einer Ausführungsform des erfindungsgemäßen Verfahrens ist die die Molybdän-basierte Verbindung anionisch.

In einer Ausführungsform erfindungsgemäßen Verfahrens enthält die Molybdän-basierte Verbindung ein oder mehrere Carbonyl-Liganden, bevorzugt ein bis sechs, besonders bevorzugt zwei bis fünf.

In einer Ausführungsform erfindungsgemäßen Verfahrens weist die Molybdän-basierte Verbindung einen weiteren Liganden (L) ungleich des Carbonyl-Liganden auf, wobei folgender funktioneller Zusammenhang zwischen dem Carbonyl-Liganden der Molybdän-basierten Verbindung mit in Summe 6 Liganden und dem Ligand (L) der Molybdän-basierten Verbindung für einkernige Molybdän-basierte Verbindungen typischerweise resultiert:

Mo(CO)₆₋ₓLₓ.

In einer Ausführungsform erfindungsgemäßen Verfahrens ist der Ligand (L) eine oder mehrere Verbindung(en) und wird ausgewählt aus der Gruppe bestehend aus Hydrido, wie beispielsweise (H),Halogenid, wie beispielsweise F, Cl, Br und I, Pseudohalogenid, wie z.besipielsweise CN, N₃, OCN, NCO, CNO, SCN, NCS und SeCN, anorganischen N-Liganden, wie beispielsweise NC, NO, NO₂, NO₃, NH₂, NCH₃, NCCH₃ und NCCF₃, Pseudochalkogenide, Carboxylate, wie beispielsweise OTf, OAc und HCOO, sonstige anorganische Anionen, wie beispielsweise OH und HSO₄, Allylverbindungen wie beispielsweise η³-C₃H₅, Diene, wie beispielsweise Butadiene (C₄C₆), cyclische C5-Liganden, wie beispielsweise Cyclopentadienyl (Cp, η⁵-C₅H₅) und Pentamethylcyclopentadienyl (Cp*, η⁵-C₅Me₅), Alkylverbindungen, Arylverbindungen, Fischer-Carbene, wie beispielsweise C(Ph)(Ph) (als Fischer-Carben), C(OMe)(Ph) (als Fischer-Carben), C(OEt)(NHPh) (als Fischer-Carben), NHC-Carbene, wie beispielsweise 1,3-Dimesitylimidazol-2-ylidene (IMes, NHC-Carben) und 1,3-Bis(2,4,6-trimethylphenyl)-4,5-dihydroimidazol-2-ylidene (SIMes, NHC-Carben), Amine, wie beispielsweise NH₃, Ethylendiamin, Diethylentriamin, Anilin, Pyridin, 2,2'-Bipyridin und Terpyridin, Imine, Aminophosphine, wie beispielsweise PNP, PDI und PBP, Phosphine, wie beispielsweise PPh₃, PMe₃, PEt₃, PBu₃, PH₃, P(OMe)₃ und P(OEt)₃, Phosphite, PEP-Pinzetten-Liganden mit E = B und N sowie Ether, wie beispielsweise Diethylether, THF und 2-Me-THF.

In einer bevorzugten Ausführungsform erfindungsgemäßen Verfahrens ist der Ligand (L) eine oder mehrere Verbindung(en) und wird ausgewählt aus der Gruppe bestehend aus H, F, Cl, Br, I, CN, NC, SCN, N₃, NO₂, NO₃, NH₂, OTf, OAc, OH, HSO₄, η³-C₃H₅), Butadiene (C₄C₆), Cyclopentadienyl (Cp, η³-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), NH₃, Ethylendiamin, Diethylentriamin, Tetramethylethylendiamin, Anilin, Pyridin, 2,2'-Bipyridin, PPh₃, PMe₃, PEt₃, PBu₃, PH₃, P(OMe)₃, P(OEt)₃, Diethylether, THF und 2-Me-THF, bevorzugt H, F, Cl, Br, I, CN, NC, SCN, N₃, NO₂, NO₃, NH₂, OTf, OAc, η³-C₃H₅, Butadiene (C₄C₆), Cyclopentadienyl (Cp, η³-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), Ethylendiamin, Diethylentriamin, Tetramethylethylendiamin, Anilin, Pyridin, 2,2'-Bipyridin, PPh₃ und PMe₃, besonders bevorzugt H, F, Cl, Br, I, CN, NC, SCN, N₃, OTf, Cyclopentadienyl (Cp, η⁵-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben) und 1,3-Bis(2,4,6-trimethylphenyl)-4,5-dihydroimidazol-2-ylidene (SIMes, NHC-Carben), ganz besonders bevorzugt Cl, Br und Pentamethylcyclopentadienyl (Cp*, η⁵-C₅Me₅).

In einer Ausführungsform des erfindungsgemäßen Verfahrens enthält das Katalysatorsystem eine zusätzliche Lewis Säure.

Lewis Säuren enthalten als Lewis-saures Zentrum ein oder mehrere koordinativ ungesättigte Metallatome, wie z.B. Aluminium, Zinn, Zink, Bismut, Vanadium, Chrom, Molybdän, Wolfram, Eisen, Kobalt, Nickel, Rhodium, Iridium, Palladium, Platin, Kupfer oder Zink. Aber auch das Halbmetall Bor bildet das Lewis-saure Zentrum von Lewis-Säuren. Koordinativ ungesättigte Lewis-saure Zentren zeichnet sich dadurch aus, dass nukleophile Moleküle daran binden können. Koordinativ ungesättigte Lewis-saure Zentren kann bereits in der als Katalysator eingesetzten Verbindung vorhanden sein oder bildet sich in der Reaktionsmischung, z.B. durch Abspaltung eines schwach gebundenen nukleophilen Moleküls.

In einer Ausführungsform des erfindungsgemäßen Verfahrens wird Mo(V)-chlorid gleichzeitig als Lewis Säure und Präkursor für die Generierung eines erfindungsgemäßen Katalysator-Systems eingesetzt (vgl. erfindungsgemäßes Beispiel 14). Unter den reduktiven Bedingungen wie sie beispielsweise unter typischen erfindungsgemäßen Reaktionsbedingungen, wie beispielsweise Druck, CO-reiche Atmosphäre, Reaktionstemperaturen bis zu 150 °C, organische und nicht protische Dispersionsmittel, herrschen, kann ein Teil von Mo(V) zu Mo(0) reduziert und bildet dabei die erfindungsgemäßen Molybdän-Komponente des Katalysator-Systems der Form [Mo(CO)ₙ(Cl)ₘ]^{m-}. Der restliche Anteil an Mo(V)-chlorid dient als erfindungsgemäße Lewis Säure. Zur Reduktion von Molybdänchloriden in Anwesenheit von CO zur Darstellung von Mo(CO)-Komplexen sei auf die Offenbarung in Georg Brauer (Hrsg.) u. a.: Handbuch der Präparativen Anorganischen Chemie. 3., umgearbeitete Auflage. Band III, Ferdinand Enke, Stuttgart 1981, ISBN 3-432-87823-0, Seite 1634 ff verwiesen.

In einer weiteren Ausführungsform des erfindungsgemäßen Verfahrens kann die Synthese der Katalysator-Systeme auch im Reaktionsgemisch selbst und/oder unter Reaktionsbedingungen erfolgen. Diese Vorgehensweise wird als zur *in-situ* Generierung eines Katalysator-Systems bezeichnet und wurde im erfindungsgemäßen Beispiel 15 durchgeführt.

In einer Ausführungsform des erfindungsgemäßen Verfahrens ist die Lewis-Säure kationisch oder ladungsneutral, bevorzugt kationisch.

In einer bevorzugten Ausführungsform des erfindungsgemäßen Verfahrens ist die Lewis-Säure kationisch.

In einer Ausführungsform des erfindungsgemäßen Verfahrens ist die kationische Lewis-Säure ein unsubstituiertes Dicyclopentadienyl-Metall-Kation, ein substituiertes Dicyclopentadienyl-Metall-Kation, ein unsubstituiertes Metall-Porphyrin-Kation, ein substituiertes Metall-Porphyrin-Kation, ein unsubstituiertes Metall-Salen-Kation, ein substituiertes Metall-Salen-Kation, ein unsubstituiertes Metall-Salphen-Kation und/oder ein und substituiertes Metall-Salphen-Kation.

Die Lewis Säuren können einfache anorganische, metallorganisch oder organische Verbindungen sein, wie z.B. BF₃, AlCl₃, FeCl₃, B(CH₃)₃, B(OH)₃, BPh₃, B(OR)₃, SiCl₄, SiF₄, PF₄, CO₂, SO₃ usw. sowie Addukte dieser Verbindungen. Aber auch komplexer aufgebaute Lewis Säuren, in denen das Lewis-saure Zentrum durch Komplex-Liganden koordiniert wird, sind bekannt und kommen in einer Ausführungsform des erfindungsgemäßen Verfahren bevorzugt zum Einsatz. Die Liganden haben dabei insbesondere die Rolle durch eine partielle koordinative Absättigung des Lewis-sauren Zentrums bzw. der Lewis-sauren Zentren eine stabilisierende und/oder verstärkende Wirkung auf die Lewis-sauren Zentren haben. Dabei kann zwischen elektronischen und sterischen Effekten der Liganden-Moleküle unterschieden werden. Quadratisch-planare und quadratisch-tetraedrische Koordinationspolyeder zwischen N- und/oder O-Ligand(en) und Lewis-sauren Zentren sind dafür besonders geeignet.

Cyclopentadienyl-Metall-Komplexe, Lewis Säuren auf Basis von unsubstituierten und substituierten Porphyrin, Chlorin und Corrin-Komplexen, Lewis Säuren auf Basis von unsubstituierten und substituierten Salen, Salpn, Salan, Salalen, Salph, Salphen und Salqu-Komplexen, Lewis Säuren auf Basis von von unsubstituierten und substituierten Pinzetten-Komplexen, Lewis Säuren auf Basis von von unsubstituierten und substituierten Pinzetten-Diiminopyridin Komplexe sind in einer Ausführungsform des erfindungsgemäßen Verfahrens als Lewis Säure besonders bevorzugt.

Cyclopentadienyl-Metall-Komplexe, welche Komplexe zwischen einem oder mehreren Metallen und einem oder mehreren Cyclopentadienyl-Derivat-Liganden (C₅R₅⁻) sind, sind dadurch gekennzeichnet das so genannte η⁵ Metall-Lagnd-Bindungen vorliegen. In einer Ausführungsform des erfindungsgemäßen Verfahrens sind Cyclopentadienyl-Metall-Komplexe (Cp-Komplexe), Pentamethylcyclopentadienyl-Metall-Komplexe (Cp*-Komplexe), Dicyclopentadienyl-Metall-Komplexe ((Cp)2-Komplexe) und Di-Pentamethyl-cyclopentadienyl-Metall-Komplexe ((Cp*)2-Komplexe) als Lewis Säuren geeignet. Neben diesen unsubstituierten Cp-Ringen und Cp*-Ring-Systemen sind aber auch alle anderen erdenklichen Substitutionsmuster möglich. In einer Ausführungsform des erfindungsgemäßen Verfahrens ist der Dicyclopentadienyl-Metall Komplex gleich [Cp₂Ti]⁺ und/oder [Cp₂Ti(L)₂]⁺ mit L = THF, Et₂O, PO und/oder EO.

Lewis Säuren auf Basis von unsubstituierten und substituierten Porphyrin, Chlorin und Corrin-Komplexen mit dem Lewis-sauren Zentrum ausgewählt aus der Gruppen von Aluminium, Zinn, Zink, Bismut, Vanadium, Chrom, Molybdän, Mangan, Wolfram, Eisen, Kobalt, Nickel, Rhodium, Iridium, Indium, Cer, Lanthan, Yttrium, Gadolinium, Palladium, Platin, Kupfer und Zink. Alle denkbaren Substitutionsmuster sind möglich. In einer Ausführungsform des erfindungsgemäßen Verfahrens sind die Substituenten unabhängig voneinander ausgewählt aus der Gruppe von Wasserstoff (-H), Methyl, tert. Butyl, Methoxy, Phenyl, 4-Methylphenyl, 4-Methoxyphenyl, 4-Chlorphenyl, 4-Bromphenyl, 4-Carboxylphenyl, 3,5-Dimethoxyphenyl, 2-Pyridyl, 4-Pyridyl und N-Methyl-4-pyridyl, bevorzugt tert. Butyl, Methoxy, Phenyl, 4-Methylphenyl, 4-Methoxyphenyl, 4-Chlorphenyl oder 3,5-Dimethoxyphenyl, ganz besonders bevorzugt Phenyl.

Lewis Säuren auf Basis von unsubstituierten und substituierten Salen, Salpn, Salan, Salalen, Salph, Salphen und Salqu-Komplexen mit dem Lewis-sauren Zentrum ausgewählt aus der Gruppen von Aluminium, Zinn, Zink, Bismut, Vanadium, Chrom, Molybdän, Mangan, Wolfram, Eisen, Kobalt, Nickel, Rhodium, Iridium, Indium, Cer, Lanthan, Yttrium, Gadolinium, Palladium, Platin, Kupfer und Zink. Alle denkbaren Substitutionsmuster sind möglich. In einer Ausführungsform des erfindungsgemäßen Verfahrens sind die Substituenten unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt ausgewählt aus der Gruppe von Wasserstoff (-H), Methyl oder tert. Butyl, ganz besonders bevorzugt tert. Butyl.

Lewis Säuren auf Basis von von unsubstituierten und substituierten Pinzetten-Komplexen (engl. pincers complexes) mit dem Lewis-sauren Zentrum ausgewählt aus der Gruppen von Aluminium, Zinn, Zink, Bismut, Vanadium, Chrom, Molybdän, Mangan, Wolfram, Eisen, Kobalt, Nickel, Ruthenium, Rhodium, Iridium, Indium, Cer, Lanthan, Yttrium, Gadolinium, Palladium, Platin, Kupfer und Zink. In einer Ausführungsform des erfindungsgemäßen Verfahrens weisen die Liganden dieser Pinzetten-Komplexe die allgemeinen Form A-(organischer Linker)-E-(organischer Linker)-A, wobei A ausgewählt aus der Gruppe der Elemente P, N und S ist und wobei E ausgewählt aus der Gruppe der Elemente C, B und N ist, und sind tridentat.

Lewis Säuren auf Basis von von unsubstituierten und substituierten Pinzetten-Diiminopyridin Komplexe (kurz: DIP-Komplexe) mit dem Lewis-sauren Zentrum ausgewählt aus der Gruppen von Aluminium, Zinn, Zink, Bismut, Vanadium, Chrom, Molybdän, Mangan, Wolfram, Eisen, Kobalt, Nickel, Ruthenium, Rhodium, Iridium, Indium, Cer, Lanthan, Yttrium, Gadolinium, Palladium, Platin, Kupfer und Zink. In einer Ausführungsform des erfindungsgemäßen Verfahrens weisen die Liganden dieser DIP-Komplexe die Form von Diiminopyridin und dessen Derivate auf.

In einer Ausführungsform des erfindungsgemäßen Verfahrens weist das Katalysatorsystem die Struktur (I), (II), (III), (IV), (V), (VI), und/oder (VII) auf: 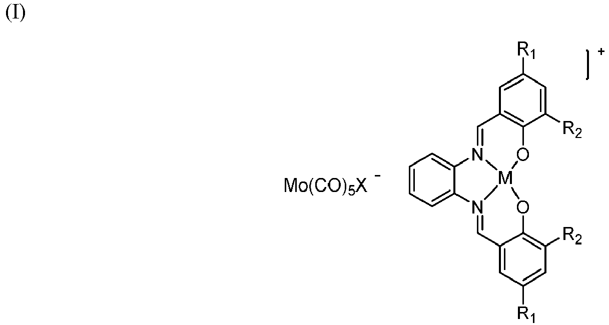
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl;

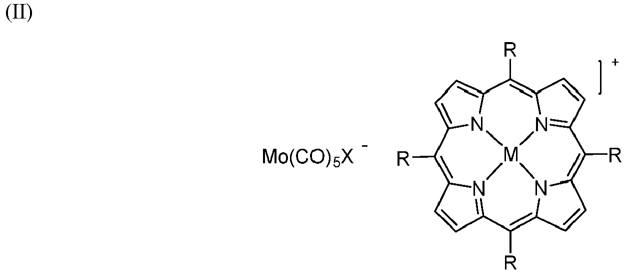

mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R ausgewählt aus der Gruppe von Wasserstoff (-H), Methyl, tert. Butyl, Methoxy, Phenyl, 4-Methylphenyl, 4-Methoxyphenyl, 4-Chlorphenyl, 4-Bromphenyl, 4-Carboxylphenyl, 3,5-Dimethoxyphenyl, 2-Pyridyl, 4-Pyridyl und N-Methyl-4-pyridyl, bevorzugt tert. Butyl, Methoxy, Phenyl, 4-Methylphenyl, 4-Methoxyphenyl, 4-Chlorphenyl oder 3,5-Dimethoxyphenyl, ganz besonders bevorzugt Phenyl.

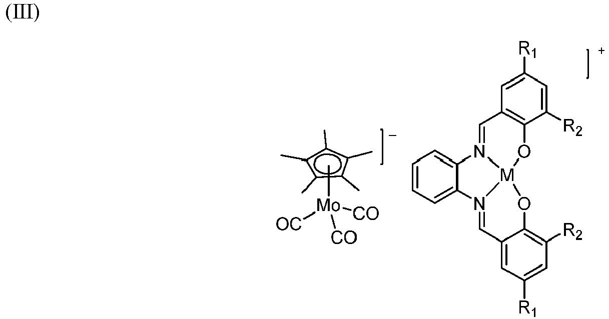

mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl;

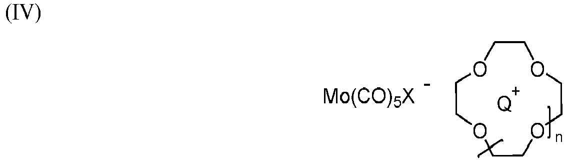

mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit Q = Li, Na, K, Rb, Cs, Cu oder Ag, bevorzugt Li, Na, K oder Rb, ganz besonders bevorzugt K;
mit n = 1-5, bevorzugt 2-5, ganz besonders bevorzugt 3; 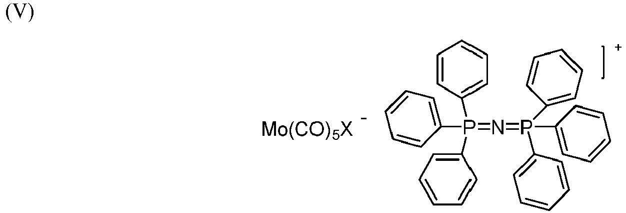
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;

(VI) Q[Mo(CO)₅]

mit Q = Li, Na, K, Rb, Cs, Cu oder Ag, bevorzugt Li, Na, K oder Rb, ganz besonders bevorzugt Na;
und /oder (VII) 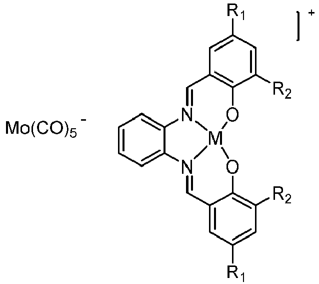
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl.

In einer bevorzugten Ausführungsform des erfindungsgemäßen Verfahrens weist das Katalysatorsystem die Struktur (VIII), (IX), (X), (XI), (XII), (XIII), (XIV), (XV) und/oder (XVI) auf:

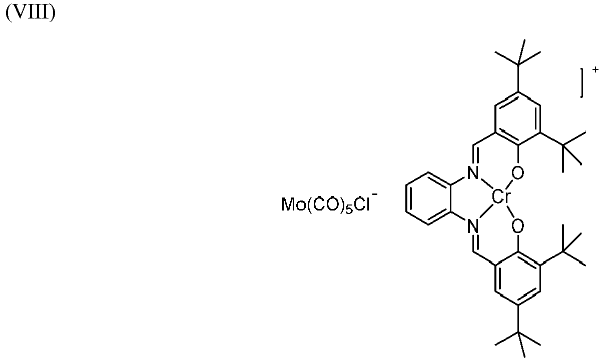

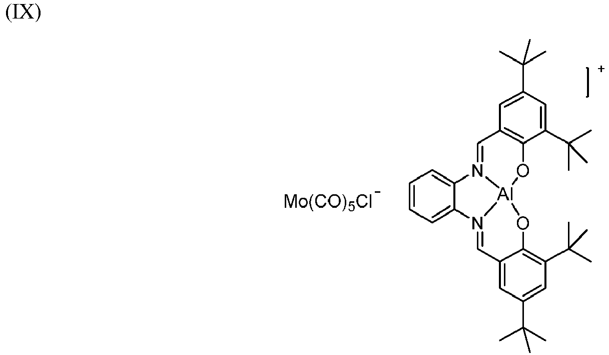

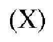

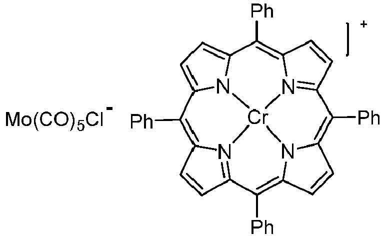

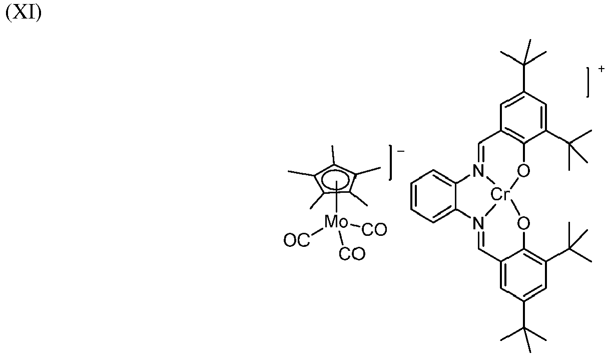

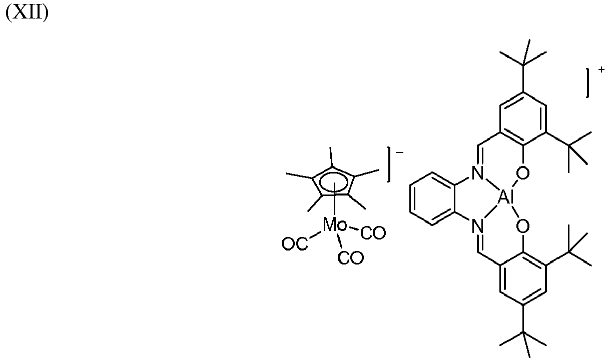

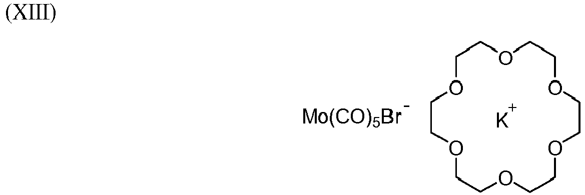

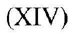

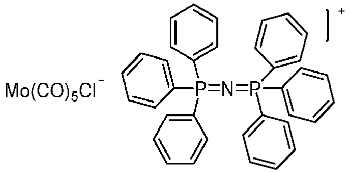

(XV) Na[Mo(CO)₅]

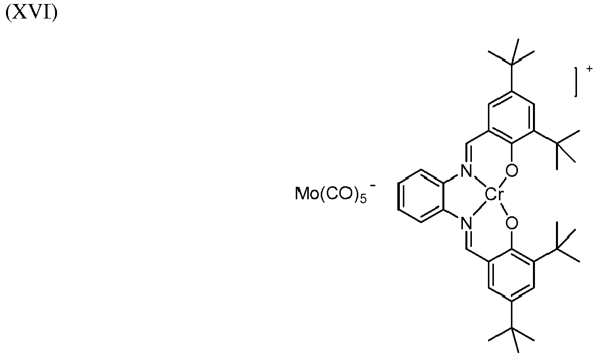

Das erfindungsgemäße Epoxid kann ein Epoxid mit 2-45 Kohlenstoffatomen sein. In einer bevorzugten Ausführungsform des Verfahrens wird das Epoxid ausgewählt aus mindestens einer Verbindung der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, Epoxide von C6-C22 α-Olefinen, wie 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Cyclopentenoxid, Cyclohexenoxid, Cycloheptenoxid, Cyclooctenoxid, Styroloxid, Methylstyroloxid, Pinenoxid, Allylglycedylether, Vinylcyclohexenoxid, Cyclooctadienmonoepoxid, Cyclododecatrienmono-epoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidylether.

In einer besonders bevorzugten Ausführungsform des Verfahrens ist das Epoxid Ethylenoxid und/oder Propylenoxid.

In einer Ausführrungsform des erfindungsgemäßen Verfahrens wird das Carbonylierungsverfahren Gegenwart eines Suspensionsmittels, bevorzugt eines ein aprotischen Suspensionsmittel durchgeführt.

Die erfindungsgemäß eingesetzten Suspensionsmittel enthalten keine H-funktionellen Gruppen. Als Suspensionsmittel sind alle polar-aprotischen, schwach polar-aprotischen und unpolaraprotischen Lösungsmittel geeignet, die jeweils keine H-funktionellen Gruppen enthalten. Als Suspensionsmittel können auch eine Mischung aus zwei oder mehrerer dieser Suspensionsmittel eingesetzt werden. Beispielhaft seien an dieser Stelle folgende polar-aprotischen Lösungsmittel genannt: 4-Methyl-2-oxo-1,3-dioxolan (im Folgenden auch als cyclisches Propylencarbonat oder cPC bezeichnet), 1,3-Dioxolan-2-on (im Folgenden auch als cyclisches Ethylencarbonat oder cEC bezeichnet), Methylformiat, Ethylformiat, Isopropylformiat, Propylformiat, Ethylacetat, Isopropylacetat, n-Butylacetat, Oxalsäuremethylester, 2,2-Diemthoxypropan, Aceton, Methylethylketon, Acetonitril, Benzonitril, Diethylcarbonat, Diemethylcarbonat, Nitromethan, Dimethylsulfoxid, Sulfolan, Dimethylformamid, Dimethylacetamid und *N*-Methylpyrrolidon.

Zu der Gruppe der unpolar- und schwach polar-aprotischen Lösungsmittel zählen z.B. Ether, wie z.B. Dioxolan, Dioxan, Diethylether, Methyl-tert-Butylether, Ethyl-tert-Butylether, tert-Amylmethylether, Butylmethylether, Methylpropylether, Dimethylether, Diisopropylether, Ethylmethylether, Methylphenylether, Dimethoxymethan, Diethoxymethan, Diemethoxyethan (Glyme), [12]Krone-4, Cyclopentylmethylether, Triglyme, Tetraglyme, Diethylenglycoldibutylether, 2-Methyltetrahydrofuran und Tetrahydrofuran, Ester, wie z.B. Essigsäureethylester und Essigsäurebutylester, Kohlenwasserstoffe, wie z.B. Pentan, n-Hexan, Benzol und alkylierte Benzolderivate (z.B. Toluol, Xylol, Ethylbenzol) und chlorierte Kohlenwasserstoffe, wie z.B., Chloroform, Chlorbenzol, Dichlorbenzol, Fluorbenzol, Difluorbenzol, Methylenechlorid, und Tetrachlorkohlenstoff. Bevorzugt als Suspensionsmittel werden 4-Methyl-2-oxo-1,3-dioxolan, 1,3-Dioxolan-2-on, 2-Methyltetrahydrofuran, Tetrahydrofuran, Dioxane, Diemethoxyethan (Glyme), Diethylether, Ethylacetat, Methylethylketon, *N*-Methylpyrrolidon, Acetonitril, Sulfolan, Dimethylsulfoxid, Dimethylformamid, Toluol, Xylol, Ethylbenzol, Dichlorbenzol, Fluorbenzol, Chlorbenzol und Difluorbenzol sowie Mischungen zweier oder mehrerer dieser Suspensionsmittel eingesetzt, besonders bevorzugt ist 4-Methyl-2-oxo-1,3-dioxolan, 1,3-Dioxolan-2-on, 2-Methyltetrahydrofuran, Tetrahydrofuran, Diemethoxyethan (Glyme), Diethylether, Ethylacetat, Acetonitril, Toluol, Dichlorbenzol, Fluorbenzol, Chlorbenzol und Difluorbenzol sowie Mischungen zweier oder mehrerer dieser Suspensionsmittel.

In einer Ausführungsform erfindungsgemäßen Verfahrens wird das Verfahren bei einem CO-Partialdruck 60 bis 130 bara durchgeführt.

In einer Ausführungsform erfindungsgemäßen Verfahrens wird das Verfahren bei Temperaturen von 0 °C bis 200 °C bevorzugt 60-190 und besonders bevorzugt 80-180 °C durchgeführt

Ein weiterer Gegenstand der vorliegenden Erfindung sind Carbonylierungsprodukte durch Umsetzung von Epoxiden mit Kohlenstoffmonoxid nach dem erfindungsgemäßen Verfahren, wobei der molare Anteil an cyclischen Anhydriden bezogen auf das eingesetzte Epoxid weniger als 5 mol% beträgt, wobei der Anteil an cyclischen Anhydriden mittels der im Experimentalteil offenbarten ¹H-NMR Methode bestimmt wurde, wobei im Speziellen auf Beispiel 24 verwiesen sei.

Hierbei sind unter Carbonylierungsprodukten Umsetzungsprodukte von Epoxiden mit Kohlenstoffmonoxid wie beispielsweise 4-Ring-Lactone, *beta*-Butyrolacton als Reaktionsprodukt von Propylenoxid und CO und Propiolacton als Reaktionsprodukt von Ethylenoxid und CO, als auch Polyhydroxyalkanoate als Polymerisationsprodukt von 4-Ring-Lactonen, insbesondere Polyhydroxybutyrat aus *beta*-Butyrolacton und Polyhydroxypropionat aus Propiolacton sowie deren Copolymere, die neben den genannten Polyester-Wiederholungseinheiten auch Polyether-Wiederholungeinheiten aufweisen, zu verstehen. Erfindungsgemäß sind unter Carbonylierungsprodukten auch Umsetzungsprodukte von Epoxiden mit Kohlenstoffmonoxid zu entsprechenden cyclischen Esterether-Verbindungen insbesondere Dioxepanon- und Dioxocanedion-Verbindungen insbesondere 1,4-Dioxepan-5-on, 2,7-Dimethyl-1,4-dioxepan-5-on, 2,6-Dimethyl-1,4-dioxepan-5-on, 3,6-Dimethyl-1,4-dioxepan-5-on, 3,7-Dimethyl-1,4-dioxepan-5-on, 1,5-Dioxocane-2,6-dion, 3,8-Dimethyl-1,5-dioxocane-2,6-dion, 3,7-Dimethyl-1,5-dioxocane-2,6-dion und 4,8-Dimethyl-1,5-dioxocane-2,6-dion.

Hierbei kann Polyhydroxybutyrat (kurz: PHB)- 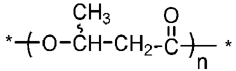 und/oder Polyhydroxypropionat (kurz: PHP) 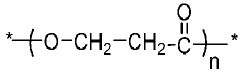 in Form von Co-Polymeren zusammen mit Polyether-Wiederholungseinheiten, 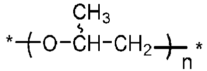 wie z.B. Polyether-Wiederholungseinheit gebildet aus PO und/oder 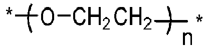 Polyether-Wiederholungseinheit gebildet aus EO, vorliegen (* = Endgruppen und/oder andere Wiederholungseinheiten).

Ein weiterer Gegenstand der vorliegenden Erfindung ist ein Verfahren zur Herstellung von Carbonylierungsfolgeprodukten, bevorzugt Polyurethanen, durch Umsetzung der erfindungsgemäßen Carbonylierungsprodukte mit Epoxiden, Polyisocyananten und/oder Polycarbonsäuren, bevorzugt mit Polyisocyanaten.

Auch die Verwendung der erfindungsgemäßen Molybdän-basierten Katalysatorsysteme für die Carbonylierung von Epoxiden sind ein Gegenstand der vorliegenden Erfindung.

In einer ersten Ausführungsform betrifft die Erfindung ein Verfahren zur Carbonylierung von Epoxiden in Gegenwart von Katalysator-Systemen, dadurch gekennzeichnet, dass das Katalysatorsystem eine Molybdän-basierte Verbindung enthält.

In einer zweiten Ausführungsform betrifft die Erfindung ein Verfahren gemäß der ersten Ausführungsform, wobei die Carbonylierung in Gegenwart von Kohlenmonoxid erfolgt.

In einer dritten Ausführungsform betrifft die Erfindung ein Verfahren gemäß der ersten oder zweiten Ausführungsform, wobei die Molybdän-basierte Verbindung ein oder mehrere Carbonyl-Liganden bevorzugt ein bis sechs Carbonyl-Liganden, besonders bevorzugt zwei bis fünf Carbonyl-Liganden enthält.

In einer vierten Ausführungsform betrifft die Erfindung ein Verfahren gemäß einer der ersten bis dritten Ausführungsform, wobei die Molybdän-basierte Verbindung einen weiteren Liganden (L) ungleich des Carbonyl-Liganden aufweist.

In einer fünften Ausführungsform betrifft die Erfindung ein Verfahren gemäß einer der ersten bis vierten Ausführungsform, wobei der Ligand (L) eine oder mehrere Verbindung(en) ist und ausgewählt wird aus der Gruppe bestehend aus H, F, Cl, Br, I, CN, NC, SCN, N₃, NO₂, NO₃, NH₂, OTf, OAc, OH, HSO₄, η³-C₃H₅, Butadiene (C₄C₆), Cyclopentadienyl (Cp, η³-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), NH₃, Ethylendiamin, Diethylentriamin, Tetramethylethylendiamin, Anilin, Pyridin, 2,2'-Bipyridin, PPh₃, PMe₃, PEt₃, PBu₃, PH₃, P(OMe)₃, P(OEt)₃, Diethylether, THF und 2-Me-THF, bevorzugt H, F, Cl, Br, I, CN, NC, SCN, N₃, NO₂, NO₃, NH₂, OTf, OAc, η³-C₃H₅, Butadiene (C₄C₆), Cyclopentadienyl (Cp, η³-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), Ethylendiamin, Diethylentriamin, Tetramethylethylendiamin, Anilin, Pyridin, 2,2'-Bipyridin, PPh₃ und PMe₃, besonders bevorzugt H, F, Cl, Br, I, CN, NC, SCN, N₃, OTf, Cyclopentadienyl (Cp, η⁵-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben) und 1,3-Bis(2,4,6-trimethylphenyl)-4,5-dihydroimidazol-2-ylidene (SIMes, NHC-Carben), ganz besonders bevorzugt Cl, Br und Pentamethylcyclopentadienyl (Cp*, η⁵-C₅Me₅).

In einer sechsten Ausführungsform betrifft die Erfindung ein Verfahren gemäß einer der ersten bis fünften Ausführungsform, wobei das Molybdän in der Molybdän-basierten Verbindung eine Oxidationsstufe von null aufweist.

In einer siebten Ausführungsform betrifft die Erfindung ein Verfahren gemäß einer der ersten bis sechsten Ausführungsform, wobei das Katalysator-System eine zusätzliche Lewis Säure enthält.

In einer achten Ausführungsform betrifft die Erfindung ein Verfahren gemäß der siebten Ausführungsform, wobei die Lewis-Säure kationisch oder ladungsneutral, bevorzugt kationisch ist.

In einer neunten Ausführungsform betrifft die Erfindung ein Verfahren gemäß der achten Ausführungsform, wobei die Lewis-Säure kationisch ist.

In einer zehnten Ausführungsform betrifft die Erfindung ein Verfahren gemäß der neunten Ausführungsform, wobei die kationische Lewis-Säure ein unsubstituiertes Dicyclopentadienyl-Metall-Kation, ein substituiertes Dicyclopentadienyl-Metall-Kation, ein unsubstituiertes Metall-Porphyrin-Kation, ein substituiertes Metall-Porphyrin-Kation, ein unsubstituiertes Metall-Salen-Kation, ein substituiertes Metall-Salen-Kation, ein unsubstituiertes Metall-Salphen-Kation und/oder ein und substituiertes Metall-Salphen-Kation ist.

In einer elften Ausführungsform betrifft die Erfindung ein Verfahren gemäß der neunten oder zehnten Ausführungsform, wobei die Molybdän-basierte Verbindung anionisch ist.

In einer zwölften Ausführungsform betrifft die Erfindung ein Verfahren gemäß einer der ersten bis elften Ausführungsform, wobei das Katalysatorsystem die Struktur (I), (II), (III), (IV), (V), (VI), und/oder (VII) aufweist: 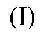 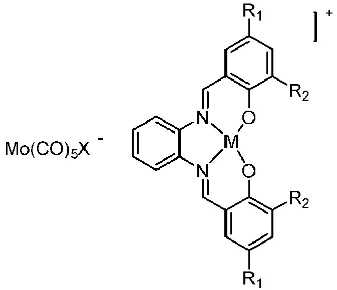
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl;

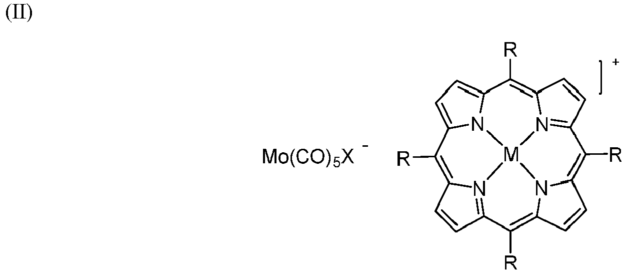

mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R ausgewählt aus der Gruppe von Wasserstoff (-H), Methyl, tert. Butyl, Methoxy, Phenyl, 4-Methylphenyl, 4-Methoxyphenyl, 4-Chlorphenyl, 4-Bromphenyl, 4-Carboxylphenyl, 3,5-Dimethoxyphenyl, 2-Pyridyl, 4-Pyridyl und N-Methyl-4-pyridyl, bevorzugt tert. Butyl, Methoxy, Phenyl, 4-Methylphenyl, 4-Methoxyphenyl, 4-Chlorphenyl oder 3,5-Dimethoxyphenyl, ganz besonders bevorzugt Phenyl.

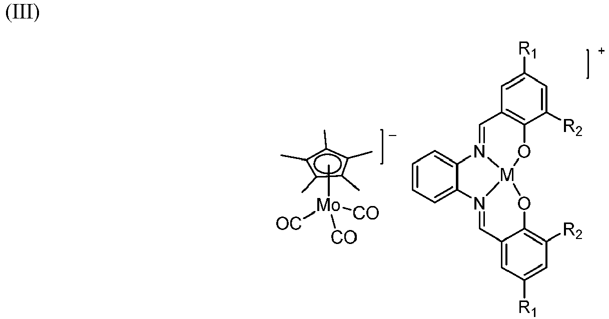

mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl;

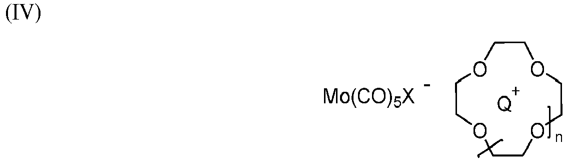

mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit Q = Li, Na, K, Rb, Cs, Cu oder Ag, bevorzugt Li, Na, K oder Rb, ganz besonders bevorzugt K;
mit n = 1-5, bevorzugt 2-5, ganz besonders bevorzugt 3; 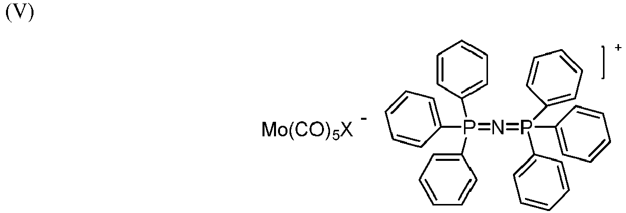
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;

(VI) Q[Mo(CO)₅]

mit Q = Li, Na, K, Rb, Cs, Cu oder Ag, bevorzugt Li, Na, K oder Rb, ganz besonders bevorzugt Na;
und /oder (VII) 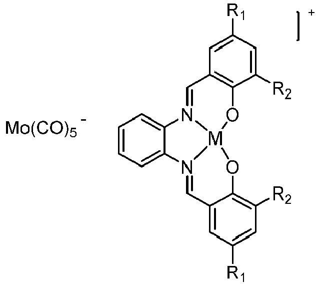
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl.

In dreizehnten Ausführungsform betrifft die Erfindung Carbonylierungsprodukte erhältlich nach mindestens einem der ersten bis zwölften Ausfürhungsform, wobei der molare Anteil an cyclischen Anhydriden bezogen auf das eingesetzte Epoxid weniger als 5 mol% beträgt, wobei der Anteil an cyclischen mittels der im Experimentalteil offenbarten 1H-NMR Methode bestimmt wurde.

In einer vierzehnten Ausführungsform betrifft die Erfindung ein Verfahren zur Herstellung von Carbonylierungsfolgeprodukten, bevorzugt Polyurethanen, durch Umsetzung der Carbonylierungsprodukte gemäß der dreizehnten Ausführungsform mit Epoxiden, Polyisocyananten und/oder Polycarbonsäuren, bevorzugt mit Polyisocyanaten.

In einer fünfzehnten Ausführungsform betrifft die Erfindung die Verwendung der Katalysator-Systeme enthaltend eine Molybdän-basierte Verbindung gemäß einem der ersten bis zwölften Ausführungsform zur Carbonylierung von Epoxiden.

In einer sechzehnten Ausführungsform betrifft die Erfindung ein Verfahren gemäß einem der ersten bis dreizehnten Ausführungsform, wobei das Katalysatorsystem die Struktur (VIII), (IX), (X), (XI), (XII), (XIII), (XIV), (XV) und/oder (XVI) aufweist:

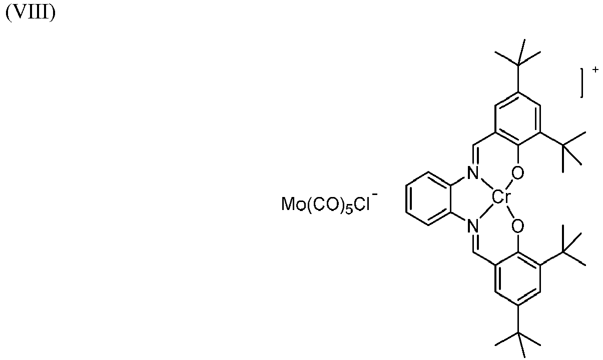

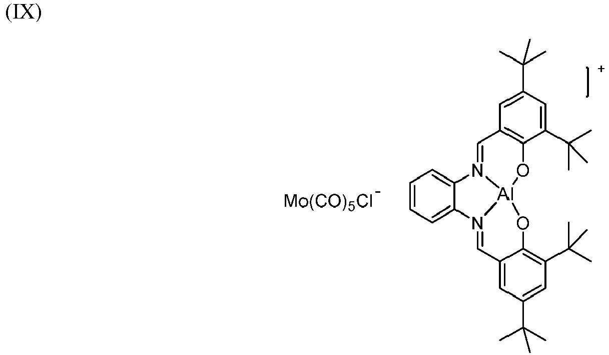

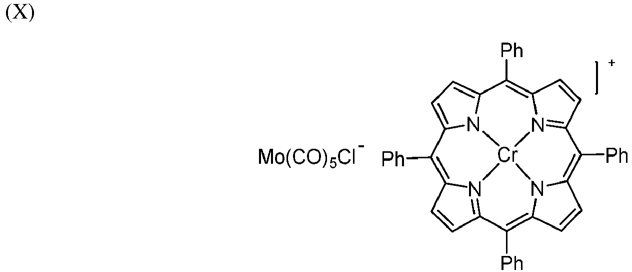

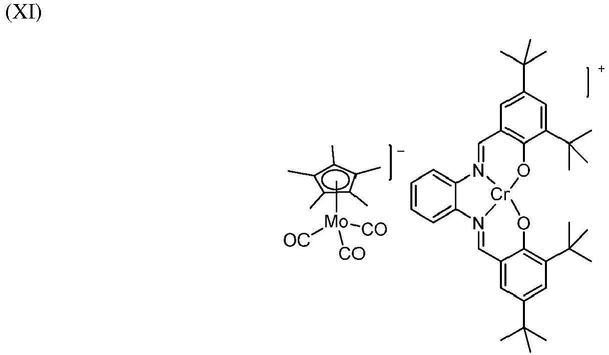

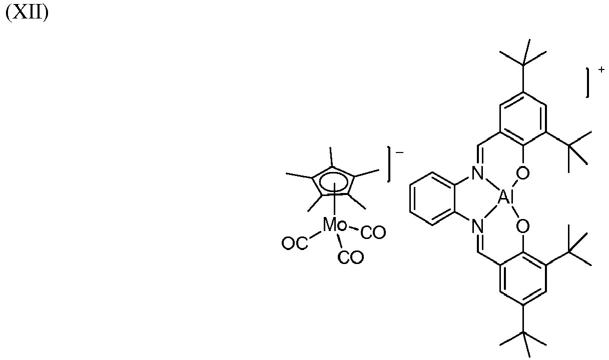

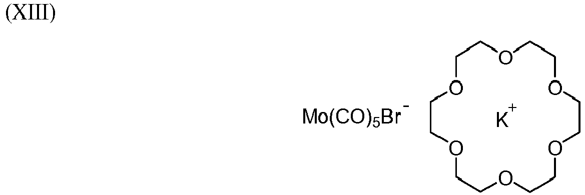

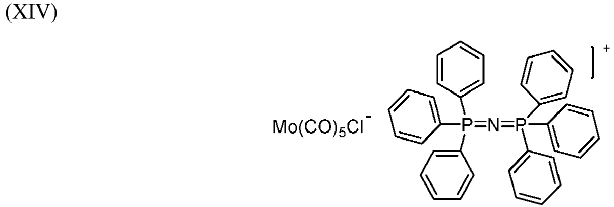

(XV) Na[Mo(CO)₅]

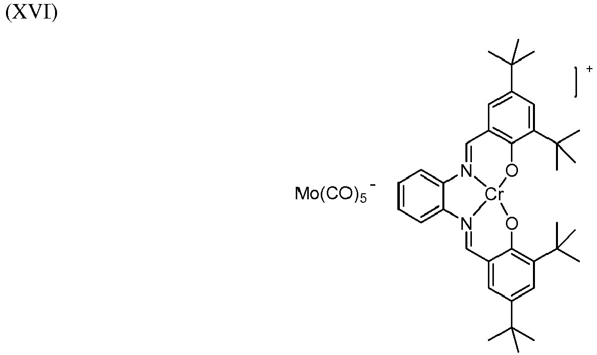

### Beispiele

### Verwendete Ausgangsstoffe:

Argon, kurz Ar, 99,998 %, Westfalen AG
Deuteriertes Chloroform, CDCl₃, 99,8 %, Eurisotop
Deuteriertes Tetrahydrofuran, THF-d₈, 99,5 %, Eurisotop
ARCOL® POLYOL 1004, kurz: PET 1004, Covestro Deutschland AG
*beta*-Butyrolacton, kurz: bBL, 98 %, Sigma Aldrich Chemie GmbH
Toluol, trocken, 99,85 %, Acros Organics
Methanol, trocken, 99,8 %, Acros Organics
Chloroform, trocken, 99,8 %, Sigma Aldrich Chemie GmbH
Diethylether, Et₂O, kurz: Ether, trocken, 99,5 %, Acros Organics
Trifluormethansulfonsäure, kurz: TfOH, 98 %, Alfa Aesar
Diisopropylethylamin, 99 %, Sigma Aldrich Chemie GmbH
Silicagel 60, kurz: Si0₂, Sigma Aldrich Chemie GmbH
Dichlormethan, kurz: DCM, trocken, 99,8 %, Acros Organics
Propiolacton, kurz: PL, 98,5 %, Ferak Berlin GmbH
1-Butanol, 99,8 %, Sigma Aldrich Chemie GmbH
3,15-Di-*tert*-butylsalicyladehyde, Sigma Aldrich Chemie GmbH
Et₂AlCl (25% in toluene), Sigma Aldrich Chemie GmbH
Chrom(II) chlorid, CrCl₂, trocken, 99,9 %, Strem Chemicals, Inc.
Molybdänhexacarbonyl, Mo(CO)₆, ≥ 99,9 %, Sigma Aldrich Chemie GmbH
Tetrahydrofuran, kurz: THF, trocken, 99,5 %, Acros Organics
*n*-Hexan, kurz: Hexan, trocken, 97 %, Acros Organics
Tetraphenylporphyrinchrom(III) chlorid, kurz: (TPP)CrCl, abcr GmbH
Cyclopentadienyllithium, kurz: Cp*Li, 97 %, Sigma Aldrich Chemie GmbH
Celite® 545, kurz: Celite, Acros Organics
*n*-Pentan, kurz: Pentan, trocken, 99 %, Acros Organics
[18]Krone-6, 99,0 %, Sigma Aldrich Chemie GmbH
Kaliumbromid, KBr, 99 %, Acros Organics
Naphthalin, kurz: Nap, 99 %, Sigma Aldrich Chemie GmbH
Natrium in Mineralöl, kurz: Na, 99,8 %, Acros Organics
Dicobaltoctacarbonyl, Co₂(CO)₈, ≥90 % (1-10 % Hexan), Sigma Aldrich Chemie GmbH
Bis(triphenylphosphoranylidene) ammonium chlorid, 97 %, Sigma Aldrich Chemie GmbH
Kohlenmonoxid, kurz: CO, 99,9 %, Paxair Deutschland GmbH
Propylenoxid, kurz: PO, 99,5 %, Sigma Aldrich Chemie GmbH
Ethylenoxid in THF, 2,5-3,3 M, Sigma Aldrich Chemie GmbH
Dimethoxyethan, kurz: DME, trocken, 99,5 %, Acros Organics
1,4-Dioxane, kurz: Dioxane, trocken, 99,8 %, Sigma Aldrich Chemie GmbH
Bortrifluorid-Etherat, BF₃ · Et₂O, kurz: BF₃, Sigma Aldrich Chemie GmbH
Molsieb 3 Å, getrocknetes, Sigma Aldrich Chemie GmbH
Magnesiumsulfat, MgSO₄, trocken, Acros Organics
Natriumsulfat, Na₂SO₄,trocken, Acros Organics
Natriumchlorid, NaCl, trocken, Acros Organics
Natriumhydroxid, NaOH, Honeywell
Dimethylformamide, kurz: DMF, Fischer Chemical
Molybdän(V) chlorid, 99,6 %, Alfa Aesar

Alle Flüssigkeiten, außer EO-Lösungen, wurden durch mehrfache Gefrier-Pump-Tau-Zyklen entgast und unter AR-Schutzgasatmosphäre gelagert. Feststoffe wurden unter Vakuum entgast und ebenfalls unter Ar-Schutzgasatmosphäre gelagert. Propylenoxid wurde durch mehrfache Gefrier-Pump-Tau-Zyklen entgast und unter Ar-Schutzgasatmosphäre und über 3 Å Molsieb bei 0 °C gelagert. Naphthalin wurde sublimiert und unter Vakuum in einer Glovebox gelagert.

### IR-Analytik:

Alle IR-Messungen wurden auf einem Bruker Alpha-PFT-IR Spektrometer durchgeführt. Falls nötig wurden die Messungen unter Ar-Schutzgas durchgeführt. Für alle gemessenen Spektren wurde eine automatische Basislinienkorrektur vorgenommen. Für weitere Details zur IR-Analytik der Produktgemische siehe unten "Quantitative IR-Analytik".

### NMR-Analytik:

NMR-Spektren wurden auf einem Bruker AV400 oder AV300 Spektrometer bei Raumtemperatur aufgenommen. 1H-NMRs wurden bei 400 bzw. 300 MHz gemessen, 13C-NMRs dementsprechend bei 100 bzw. 75 MHz. Die 1H- und 13C-NMR Signale sind gegen CHC13 bzw. TMS referenziert.

### GC-Analytik:

Gas-Chromatogramme wurden auf einem Thermo-Scientific Gerät mit einer Trace-1 VF-WAX ms II Kapillarsäule (30 m x 0.25 mm; Film-Dicke= 0.25 µm), FID und He als Trägergas aufgenommen. Temperaturprofile 40-220 °C mit 8 °C/min und 15 min iso bei 220 °C. Retentionszeiten (in min) = Naphthalin (interner Standard) 24.0, *beta*-Butyrolactone 22.5, Propylenoxid 5.1, Ethylenoxid 4.4 und Propiolacton 17.9.

### Quantitative IR-Analytik:

Zur Kalibrierung der quantitativen IR wurden Gemisch aus Polyhydroxybutyrat und Polyether mit 0-25 wt.-% Polyhydroxybutyrat-Anteil angefertigt und vermessen. Die Kalibrierungsgerade ergibt sich aus der Auftragung der relativen Absorbanz gegen den relativen Gewichtsanteil an Polyhydroxybutyrat. Die relativen Absorbanz wird aus dem Verhältnis der Absorbanz der C=O Bande zu dem der C-O Bande bestimmt. Der relative Massenanteil (in wt.-%) an Polyhydroxybutyrat- zu Polyether-Wiederholungseinheiten - in nicht flüchtigen Reaktionsrückständen (vgl. Allgemeine Versuchsvorschrift) - wurde somit wie folgt berechnen: w(Polyhydroxybutyrat) = relativen Absorbanz/0,0145. Analog wurde für Polyhydroxypropionat vorgegangen. Der relative Massenanteil (in wt.-%) an Polyhydroxypropionat- zu Polyether-Wiederholungseinheiten - in nicht flüchtigen Reaktionsrückständen (vgl. Allgemeine Versuchsvorschrift) - wurde somit wie folgt berechnen: w(Polyhydroxypropionat) = relativen Absorbanz/0,053.

### Polyether (kurz PET 1004) als Refrenzpolymer:

Als Polyether-Refrenzpolymer für die quantitative IR und NMR-Analytik wurde ARCOL® POLYOL 1004 (kurz PET 1004) der Fa. Covestro verwendet. Es handelt sich dabei um ein bifunktionelles Polyetherpolyol auf Basis von PO mit einem mittleren MW von ca. 435 g/mol.

IR: v = 1087 (C-O) cm⁻¹.
¹H NMR (CDCl₃, 7.26 ppm): 1.07-1.16 (m, CH₃), 3.14-3.92 (m, CH, CH₂);
¹³C NMR (CDCl₃, 77.16 ppm): 17.0-18.6, 65.7-67.3, 73.4-76.1 ppm

### Synthese von Polyhydroxybutyrat (kurz PHB):

Die Synthese von Polyhydroxybutyrat (kurz PHB) als Referenzpolymer für die quantitative IR und NMR-Analytik erfolgt in Anlehnung an Tetrahedron Asymmetry, 2003, 14, 3249-3252 bzw. Polym. Chem., 2014, 5, 161-168. Unter Ar-Schutzgasatmosphäre wurden 3,8 mL β-Butyrolacton und 40 mL Toluol in einen Schlenkkolben vorgelegt, mit je 0,07 mL Methanol und Trifluormethansulfonsäure versetzt und anschließend bei 30 °C für 2 h gerührt. Anschließend wurde mit 0,15 mL Diisopropylethylamin gequencht, am Rotationsverdampfer eingeengt, säulenchromatographisch aufgereinigt (stationäre Phase: SiO₂, Laufmittel: DCM/MeOH 20:1 v/v) und von Lösungsmittel befreit. Das Referenzpolymer PHB wurde final am Hochvakuum getrocknet und mittels IR und NMR analysiert.

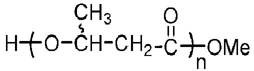

IR: v = 1731 (C=O) cm⁻¹.
¹H NMR (CDCl₃, 7.26 ppm): 5.25 (s, CH), 3.67 (s, OCH₃) 2.4-2.63 (d, CH₂), 1.25-1.28 (d, CH₃) ppm.
¹³C NMR (CDCl₃, 77.16 ppm): 20.0 (CH₃), 41.0 (CH₂), 67.8 (CH), 169.4 (C=O) ppm.

### Synthese von Polyhydroxypropionate (kurz: PHP):

Die Synthese von Polyhydroxypropionate (kurz PHP) als Referenzpolymer für die quantitative IR und NMR-Analytik erfolgt analog zu PHB. Unter Ar-Schutzgasatmosphäre wurden 2,93 mL Propiolacton und 40 mL Toluol in einen Schlenkkolben vorgelegt, mit je 0,07 mL Methanol und Trifluormethansulfonsäure versetzt und anschließend bei Raumtemperatur für 0,5 h gerührt. Anschließend wurde mit 0,15 mL Diisopropylethylamin gequencht, filtriert und mit Toluol und Diethylether gewaschen. Das Referenzpolymer PHP wurde final am Hochvakuum getrocknet und mittels IR und NMR analysiert.

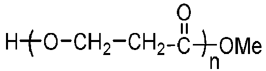

IR: v = 1725 (C=O) cm⁻¹.
¹H NMR (CDCl₃, 7.26 ppm): 2.55 (t, CH₂), 4.22 (m, CH₂) ppm.
¹³C NMR (CDCl₃, 77.16 ppm); 33.4 (CH₂), 59.9 (CH₂), 170.1 (C=O) ppm.

### Synthese des Salphen-Komplex Salz A:

Die Synthese des Salphen-Komplexes Salz A erfolgte nach Jiang et al. in Top. Catal., 2017, 60, 750-754 und eine isolierter Ausbeute von 77 % wurde erhalten.

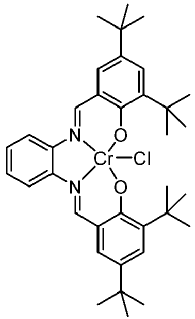

### Synthese des Salphen-Komplex Salz B:

Die Synthese des Salphen-Komplexes Salz B erfolgte nach Getzler et al. in J. Am. Chem. Soc., 2002, 124, 1174-1175 und eine isolierter Ausbeute von 91 % wurde erhalten.

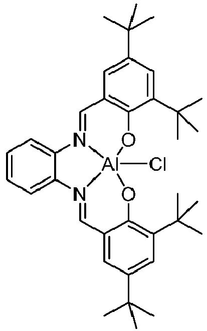

### Synthese von Katalysator-System 1 der Formel (VIII):

Unter Ar-Schutzgasatmosphäre wurden Mo(CO)₆ (0,42 g, 1,60 mmol) und Salphen-Komplex Salz A (1 g, 1,60 mmol) in einen Schlenkkolben mit Rückflusskühler vorgelegt, mit 35 mL THF versetzt und anschließend 5 h unter Rückfluss gekocht. Es wurde auf Raumtemperatur abgekühlt, filtriert und das resultierende Filtrat bis zur vollständigen Trocknung unter Vakuum eingeengt. Das Katalysator-System 1 wurde mit 87 % (1,4 g, 1,40 mmol) isolierter Ausbeute erhalten und mittels IR untersucht. Dabei wurden charakteristische Carbonyl-Banden bei 2027, 1943 und 1864 cm⁻¹ identifiziert.

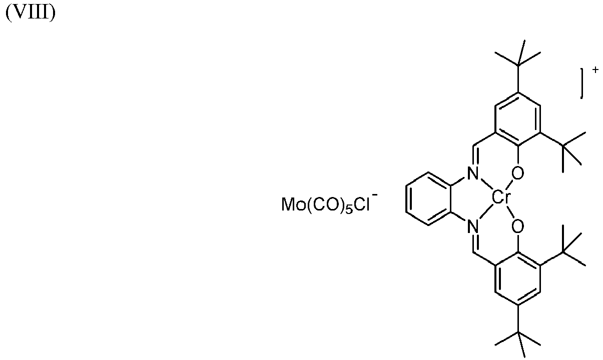

### Synthese von Katalysator-System 2 der Formel (IX):

Unter Ar-Schutzgasatmosphäre wurden Mo(CO)₆ (0,44 g, 1,67 mmol) und Salphen-Komplex Salz B (1 g, 1,67 mmol) in einen Schlenkkolben mit Rückflusskühler vorgelegt, mit 50 mL THF versetzt und anschließend 17 h unter Rückfluss gekocht. Es wurde auf Raumtemperatur abgekühlt, filtriert, das resultierende Filtrat bis zur vollständigen Trocknung unter Vakuum eingeengt, mit Hexan gewaschen und erneut getrocknet. Das Katalysator-System 2 wurde mit 36 % (0,56 g, 0,36 mmol) isolierter Ausbeute erhalten und mittels IR untersucht. Dabei wurden charakteristische Carbonyl-Banden bei 2071, 1922 und 1848 cm⁻¹ identifiziert.

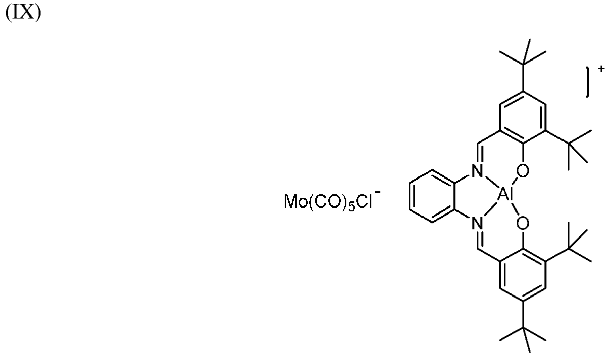

### Synthese von Katalysator-System 3 der Formel (X):

Unter Ar-Schutzgasatmosphäre wurden Mo(CO)₆ (0,095 g, 0,36 mmol) und (TPP)CrCl (1 g, 1,67 mmol) in einen mit Aluminiumfolie umwickelten Schlenkkolben mit Rückflusskühler vorgelegt, mit 40 mL THF versetzt und anschließend 3 h unter Rückfluss gekocht. Es wurde auf Raumtemperatur abgekühlt, das resultierende Reaktionsgemisch bis zur vollständigen Trocknung unter Vakuum eingeengt, zweimal mit Hexan gewaschen und erneut getrocknet. Das Katalysator-System 3 wurde mit 53 % (0,19 g, 0,17 mmol) isolierter Ausbeute erhalten und mittels IR untersucht. Dabei wurden charakteristische Carbonyl-Banden bei 2060, 1932 und 1871 cm⁻¹ identifiziert.

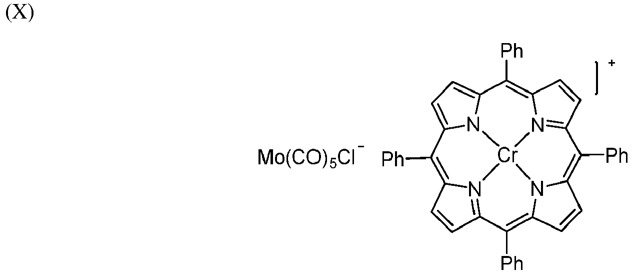

### Synthese von Katalysator-System 4 der Formel (XI):

Unter Ar-Schutzgasatmosphäre wurden Mo(CO)₆ (1,80 g, 7,03 mmol) und Cp*Li (1 g, 7,03 mmol) in einen Schlenkkolben mit Rückflusskühler vorgelegt, mit 35 mL THF versetzt und anschließend 22 h unter Rückfluss gekocht. Es wurde auf Raumtemperatur abgekühlt, das resultierende Reaktionsgemisch bis zur vollständigen Trocknung unter Vakuum eingeengt, zweimal mit Hexan gewaschen und erneut getrocknet. Das leicht gelbliche, feste Zwischenprodukt Li[Cp*Mo(CO)₃] wurde mit 86 % (1,95 g, 6,06 mmol) isolierter Ausbeute erhalten und mittels IR untersucht (charakteristische IR-Banden: v = 2060, 1932 und 1871 cm⁻¹). Anschließend wurden unter Ar-Schutzgasatmosphäre wurden Li[Cp*Mo(CO)₃] (0,96 g, 2,97 mmol) und Salphen-Komplex Salz A (1 g, 2,97 mmol) in einen Schlenkkolben vorgelegt, mit 150 mL THF versetzt und über Nacht gerührt. Das resultierende Reaktionsgemisch wurde über Celite filtriert, anschließend bis zur vollständigen Trocknung unter Vakuum eingeengt, zweimal mit kaltem Pentan gewaschen und erneut getrocknet. Das Katalysator-System 4 wurde mit 61 % (1,92 g, 1,80 mmol) isolierter Ausbeute erhalten und mittels IR untersucht. Dabei wurden charakteristische Carbonyl-Banden bei 1921 und 1868 cm⁻¹ identifiziert.

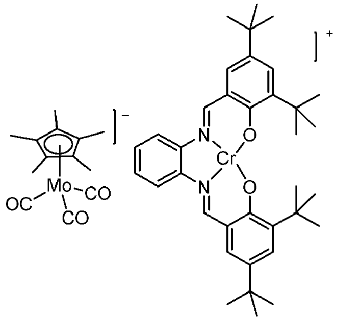

### Synthese von Katalysator-System 5 der Formel (XII):

Das Zwischenprodukt Li[Cp*Mo(CO)₃] wurde analog wie für Katalysator-System 4 hergestellt. Anschließend wurden unter Ar-Schutzgasatmosphäre wurden Li[Cp*Mo(CO)₃] (0,27 g, 0,83 mmol) und Salphen-Komplex Salz B (0,5 g, 0,83 mmol) in einen Schlenkkolben vorgelegt, mit 40 mL THF versetzt und über Nacht gerührt. Das resultierende Reaktionsgemisch wurde über Celite filtriert und anschließend bis zur vollständigen Trocknung unter Vakuum eingeengt. Das Katalysator-System 5 wurde mit 44 % (0,38 g, 0,37 mmol) isolierter Ausbeute erhalten und mittels IR untersucht. Dabei wurden charakteristische Carbonyl-Banden bei 1916 und 1865 cm⁻¹ identifiziert.

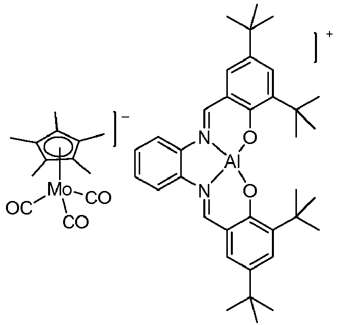

### Synthese von Katalysator-System 6 der Formel (XIII):

Die Synthese des Katalysator-Systems 6 erfolgte nach WO 2007/073225 A1 und eine isolierter Ausbeute von 87 % wurde erhalten.

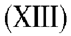

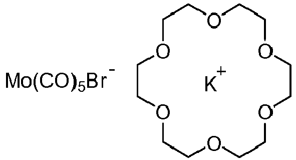

### Synthese von Katalysator-System 7 der Formel (XIV):

Unter Ar-Schutzgasatmosphäre wurden Mo(CO)₆ (0,20 g, 0,76 mmol) und (PPN)Cl (0,43 g, 0,76 mmol) in einen Schlenkkolben mit Rückflusskühler vorgelegt, mit 5 mL THF versetzt und anschließend 2 h unter Rückfluss gekocht. Es wurde auf Raumtemperatur abgekühlt, filtriert und das resultierende Filtrat auf 0 °C abgekühlt, mit Hexan versetzt und zwei Tage bei 0 °C gelagert. Anschließend wurde die flüssige Phase abdekantiert und der Rückstand mit DCM gewaschen und bis zur vollständigen Trocknung unter Vakuum eingeengt. Das Katalysator-System 7 wurde mit 54 % (0,33 g, 0,41 mmol) isolierter Ausbeute erhalten und mittels IR untersucht. Dabei wurden charakteristische Carbonyl-Banden bei 2065, 1900 und 1848 cm⁻¹ identifiziert.

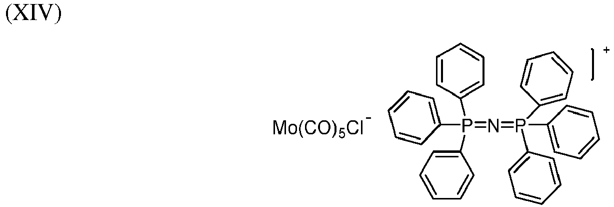

### Synthese von Katalysator-System 8 der Formel (XV):

Unter Ar-Schutzgasatmosphäre wurden frisch sublimiertes Naphthalin (3,16 g, 24,60 mmol) und fein geschnittenes Natrium (0,53 g, 23,40 mmol) in einen Schlenkkolben vorgelegt, mit 55 mL THF versetzt und anschließend zwei Tage gerührt. Die erhaltene grüne Natriumnaphthenat-Lösung wurde bei -26 °C gelagert. Anschließend wurden Mo(CO)₆ (1 g, 3,80 mmol) und 10,5 mL THF in einen Schlenkkolben vorgelegt, auf -60 °C abgekühlt und tropfenweise die zuvor präparierte Natriumnaphthenat-Lösung unter Rühren zugegeben. Nach der vollständigen Zugabe wurde langsam auf Raumtemperatur erwärmt und für weitere 24 h gerührt. Anschließend wurde das Reaktionsgemisch bis zur vollständigen Trocknung unter Vakuum eingeengt, dreimal mit Hexan gewachsen und erneut bis zur vollständigen Trocknung unter Vakuum eingeengt. Das Katalysator-System 8 (Formel: Na[Mo(CO)₅]) wurde mit 87 % (0,86 g, 3,30 mmol) isolierter Ausbeute erhalten und mittels IR untersucht. Dabei wurden charakteristische Carbonyl-Banden bei 1864 und 1733 cm⁻¹ identifiziert.

### Synthese von Katalysator-System 9 der Formel (XVI):

Unter Ar-Schutzgasatmosphäre wurden Na[Mo(CO)₅] (Katalysator-System 8, 0,1 g, 0,38 mmol) und Salphen-Komplex Salz A (0,24 g, 0,38 mmol) in einen Schlenkkolben vorgelegt, mit 10 mL THF versetzt und anschließend 22 h gerührt. Das resultierende Reaktionsgemisch wurde filtriert und das Filtrat bis zur vollständigen Trocknung unter Vakuum eingeengt. Das Katalysator-System 9 wurde mit 44 % (0,38 g, 0,0,37 mmol) isolierter Ausbeute erhalten und mittels IR untersucht. Dabei wurden charakteristische Carbonyl-Banden bei 1931 und 1864 cm⁻¹ identifiziert.

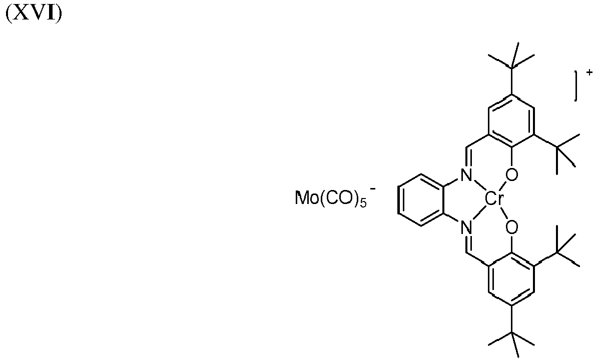

### Synthese von Katalysator-System 10 der Formel (XVII)(Vergleich):

Die Synthese des Katalysator-Systems 10 erfolgte nach Kramer et al. in Org. Lett., 2006, 8, 3709-3712 und eine isolierter Ausbeute von 68 % wurde erhalten.

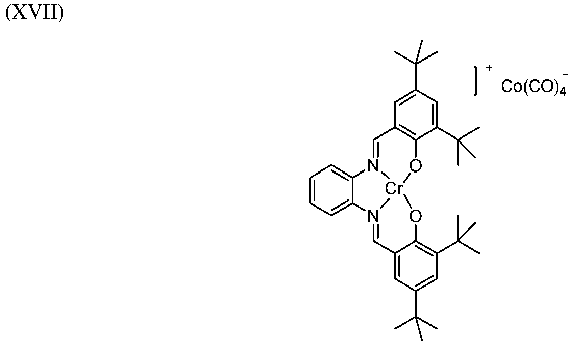

### Allgemeine Versuchsvorschrift:

Katalysator-System, Suspensionsmittel, Naphthalin als interner Standard und Epoxid wurden unter Ar-Schutzgasgegenstrom in ein Schlenkrohr eingewogen und gerührt. Das Gesamtvolumen des Gemischs betrug 2-5 mL. Dieses Gemisch wurde nun vollständig unter einem leichten CO-Gasgegenstrom in einen 10 mL-Druckreaktor überführt. Der gewünschte CO-Vordruck wurde unter Rühren eingestellt, auf Reaktionstemperatur aufgeheizt und der Anfangsdruck bei Reaktionstemperatur bestimmt. Am Ende der Reaktionszeit wurde der Enddruck bei Reaktionstemperatur bestimmt und der Druckreaktor anschließend mit Hilfe eines Wasser/Eis-Gemischs abgekühlt. Der verbleibende Restdruck wurde langsam abgelassen und unverzüglich eine Probe des Reaktionsgemischs zur Analyse entnommen. Ein Teil der Probe wurde nach Filtration über eine Kurzwegsäule mit Celite als stationäre Phase mittels NMR und GC analysiert. Ein anderer Teil der Probe wurde unter Vakuum von flüchtigen Bestandteilen befreit und mittels quantitativer IR und NMR analysiert. Die Polymerausbeute wurde gravimetrisch unter Berücksichtigung des zurückbleibenden Katalysators bestimmt. Die flüchtigen Bestandteile wurden dabei mit Hilfe einer Kühlfalle aufgefangen, Naphthalin als externer Standard zugegeben und das Gemisch ebenfalls analysiert.

### Beispiel 1: Carbonylierung von Propylenoxid unter Einsatz von Katalysator-System 1

Der Versuch wurde wie in der allgemeinen Versuchsvorschrift beschrieben durchgeführt. 1 mol% des Katalysator-Systems 1, THF als Suspensionsmittel, Naphthalin als interner Standard (0,009 mmol) und PO (0,924 mmol, 0,9 M) wurden eingesetzt. Die Reaktion wurde bei 120 °C, 80 bar Anfangsdruck und 23 h Reaktionszeit durchgeführt. Ein PO-Umsatz von 85 % wurde mittels NMR im Reaktionsgemisch bestimmt. Mittels GC-Analyse des Reaktionsgemischs wurde die Bildung von 3 % *beta*-Butyrolacton nachgewiesen. Die nichtflüchtigen Bestandteile wurden nicht isoliert und nicht gesondert analysiert.

### Beispiel 2: Carbonylierung von Propylenoxid unter Einsatz von Katalysator-System 1

Der Versuch wurde wie in der allgemeinen Versuchsvorschrift beschrieben durchgeführt. 1 mol% des Katalysator-Systems 1, THF als Suspensionsmittel, Naphthalin als interner Standard (1 mmol) und PO (10 mmol, 1,8 M) wurden eingesetzt. Die Reaktion wurde bei 120 °C, 130 bar Anfangsdruck und 48 h Reaktionszeit durchgeführt. Es wurde ein Umsatz an CO beobachtet, die Differenz zwischen Anfangs- und Enddruck betrug 20 bar. Die nichtflüchtigen Bestandteile des Reaktionsgemisches wurden isoliert und analysiert. Es wurde eine Polymerausbeute von 14 % mit einem Anteil von 17 wt.-% PHB im polymeren Produkt bestimmt.

### Beispiel 3: Carbonylierung von Propylenoxid unter Einsatz von Katalysator-System 1

Der Versuch wurde wie in der allgemeinen Versuchsvorschrift beschrieben durchgeführt. 2 mol% des Katalysator-Systems 1, THF als Suspensionsmittel, Naphthalin als interner Standard (0,09 mmol) und PO (0,924 mmol, 0,9 M) wurden eingesetzt. Die Reaktion wurde bei 100 °C, 60 bar Anfangsdruck und 46 h Reaktionszeit durchgeführt. Ein PO-Umsatz von 65 % wurde mittels NMR im Reaktionsgemisch bestimmt. Die nichtflüchtigen Bestandteile des Reaktionsgemisches wurden isoliert und analysiert. Es wurden ein Gehalt an 17 wt.-% PHB im polymeren Produkt bestimmt.

### Beispiel 4: Carbonylierung von Propylenoxid unter Einsatz von Katalysator-System 1

Der Versuch wurde wie in der allgemeinen Versuchsvorschrift beschrieben durchgeführt. 2 mol% des Katalysator-Systems 1, THF als Suspensionsmittel, Naphthalin als interner Standard (0,09 mmol) und PO (0,924 mmol, 0,9 M) wurden eingesetzt. Die Reaktion wurde bei 150 °C, 100 bar Anfangsdruck und 23 h Reaktionszeit durchgeführt. Ein PO-Umsatz von 94 % wurde mittels NMR im Reaktionsgemisch bestimmt. Die nichtflüchtigen Bestandteile des Reaktionsgemisches wurden isoliert und analysiert. Es wurde eine Polymerausbeute von 50 % mit einem Anteil von 45 wt.-% PHB im polymeren Produkt bestimmt.

### Beispiel 5: Carbonylierung von Ethylenoxid unter Einsatz von Katalysator-System 1

Der Versuch wurde wie in der allgemeinen Versuchsvorschrift beschrieben durchgeführt. 1 mol% des Katalysator-Systems 1, THF als Suspensionsmittel, Naphthalin als interner Standard (0,9 mmol) und EO (9,92 mmol, 2 M) wurden eingesetzt. Die Reaktion wurde bei 150 °C, 100 bar Anfangsdruck und 87 h Reaktionszeit durchgeführt. Ein EO-Umsatz von 98 % wurde mittels NMR im Reaktionsgemisch bestimmt. Die nichtflüchtigen Bestandteile des Reaktionsgemisches wurden isoliert und analysiert. Es wurde eine Polymerausbeute von > 99 % mit einem Anteil von 3 wt.-% PHP im polymeren Produkt bestimmt. Die Massenbilanz bezogen auf EO betrugt > 99 %.

### Beispiel 6: Carbonylierung von Propylenoxid unter Einsatz von Katalysator-System 1

Der Versuch wurde wie in der allgemeinen Versuchsvorschrift beschrieben durchgeführt. 1 mol% des Katalysator-Systems 1, DME als Suspensionsmittel, Naphthalin als interner Standard (0,2 mmol) und PO (2 mmol, 0,9 M) wurden eingesetzt. Die Reaktion wurde bei 120 °C, 80 bar Anfangsdruck und 19 h Reaktionszeit durchgeführt. Die nichtflüchtigen Bestandteile des Reaktionsgemisches wurden isoliert und analysiert. Es wurde ein Anteil von 39 wt.-% PHB im polymeren Produkt bestimmt.

### Beispiel 7: Carbonylierung von Propylenoxid unter Einsatz von Katalysator-System 1

Der Versuch wurde wie in der allgemeinen Versuchsvorschrift beschrieben durchgeführt. 2 mol% des Katalysator-System 1, DME als Suspensionsmittel, Naphthalin als interner Standard (0,2 mmol) und PO (2 mmol, 0,9 M) wurden eingesetzt. Die Reaktion wurde bei 120 °C, 80 bar Anfangsdruck und 19 h Reaktionszeit durchgeführt. Ein PO-Umsatz von 69 % wurde mittels NMR im Reaktionsgemisch bestimmt. Die nichtflüchtigen Bestandteile des Reaktionsgemisches wurden isoliert und analysiert. Es wurde eine Polymerausbeute von 7 % mit einem Anteil von 14 wt.-% PHB im polymeren Produkt bestimmt.

### Beispiel 8: Carbonylierung von Propylenoxid unter Einsatz von Katalysator-System 1

Der Versuch wurde wie in der allgemeinen Versuchsvorschrift beschrieben durchgeführt. 0,1 mol% des Katalysator-System 1, DME als Suspensionsmittel, Naphthalin als interner Standard (1 mmol) und PO (10 mmol, 1,8 M) wurden eingesetzt. Die Reaktion wurde bei 150 °C, 70 bar Anfangsdruck und 65 h Reaktionszeit durchgeführt. Es wurde ein Umsatz an CO beobachtet, die Differenz zwischen Anfangs- und Enddruck betrug 13 bar. Ein PO-Umsatz von 41 % und die Bildung von < 1 % Aceton wurden mittels NMR im Reaktionsgemisch bestimmt. Die nichtflüchtigen Bestandteile des Reaktionsgemisches wurden isoliert und analysiert. Es wurde eine Polymerausbeute von 8 % mit einem Anteil von 58 wt.-% PHB im polymeren Produkt bestimmt.

### Beispiel 9: Carbonylierung von Propylenoxid unter Einsatz von Katalysator-System 1

Der Versuch wurde wie in der allgemeinen Versuchsvorschrift beschrieben durchgeführt. 1 mol% des Katalysator-System 1, DME als Suspensionsmittel, Naphthalin als interner Standard (1 mmol) und PO (10 mmol, 1,8 M) wurden eingesetzt. Die Reaktion wurde bei 150 °C, 70 bar Anfangsdruck und 65 h Reaktionszeit durchgeführt. Es wurde ein Umsatz an CO beobachtet, die Differenz zwischen Anfangs- und Enddruck betrug 15 bar. Ein PO-Umsatz von 67 % und die Bildung von 1,5 % Aceton wurden mittels NMR im Reaktionsgemisch bestimmt. Die nichtflüchtigen Bestandteile des Reaktionsgemisches wurden isoliert und analysiert. Es wurde eine Polymerausbeute von 45 % mit einem Anteil von 28 wt.-% PHB im polymeren Produkt bestimmt.

### Beispiel 10: Carbonylierung von Ethylenoxid unter Einsatz von Katalysator-System 1

Der Versuch wurde wie in der allgemeinen Versuchsvorschrift beschrieben durchgeführt. 1 mol% des Katalysator-Systems 1, ein 2:1-Gemisch (v/v) aus THF und DME als Suspensionsmittel, Naphthalin als interner Standard (0,9 mmol) und EO (9,92 mmol, 2 M) wurden eingesetzt. Die Reaktion wurde bei 120 °C, 70 bar Anfangsdruck und 65 h Reaktionszeit durchgeführt. Ein EO-Umsatz von 97 % wurde mittels NMR im Reaktionsgemisch bestimmt. Die nichtflüchtigen Bestandteile des Reaktionsgemisches wurden isoliert und analysiert. Es wurde eine Polymerausbeute von > 99 % mit einem Anteil von 2 wt.-% PHP im polymeren Produkt bestimmt. Die Massenbilanz bezogen auf EO betrugt > 99 %.

### Beispiel 11: Carbonylierung von Propylenoxid unter Einsatz von Katalysator-System 1

Der Versuch wurde wie in der allgemeinen Versuchsvorschrift beschrieben durchgeführt. 0,4 mol% des Katalysator-System 1, kein Suspensionsmittel, Naphthalin als interner Standard (1 mmol) und PO (10 mmol) wurden eingesetzt. Die Reaktion wurde bei 120 °C, 80 bar Anfangsdruck und 19 h Reaktionszeit durchgeführt. Ein PO-Umsatz von 89 % wurde mittels NMR im Reaktionsgemisch bestimmt. Die nichtflüchtigen Bestandteile des Reaktionsgemisches wurden isoliert und analysiert. Es wurde eine Polymerausbeute von > 8 % mit einem Anteil von 4 wt.-% PHB im polymeren Produkt bestimmt.

### Beispiel 12: Carbonylierung von Propylenoxid unter Einsatz von Katalysator-System 2

Der Versuch wurde wie in der allgemeinen Versuchsvorschrift beschrieben durchgeführt. 1 mol% des Katalysator-System 2, DME, Naphthalin als interner Standard (1 mmol) und PO (10 mmol, 1,8 M) wurden eingesetzt. Die Reaktion wurde bei 150 °C, 70 bar Anfangsdruck und 65 h Reaktionszeit durchgeführt. Ein PO-Umsatz von 54 % und die Bildung von 2,5 % Aceton wurden mittels NMR im Reaktionsgemisch bestimmt. Die nichtflüchtigen Bestandteile des Reaktionsgemisches wurden isoliert und analysiert. Es wurde eine Polymerausbeute von > 8 % mit einem Anteil von 16 wt.-% PHB im polymeren Produkt bestimmt.

### Beispiel 13: Carbonylierung von Propylenoxid unter Einsatz von Katalysator-System 3

Der Versuch wurde wie in der allgemeinen Versuchsvorschrift beschrieben durchgeführt. 1 mol% des Katalysator-System 3, THF, Naphthalin als interner Standard (0,4 mmol) und PO (4 mmol, 0,9 M) wurden eingesetzt. Die Reaktion wurde bei 150 °C, 75 bar Anfangsdruck und 21 h Reaktionszeit durchgeführt. Ein PO-Umsatz von 78 % und die Bildung von 2,3 % Aceton wurden mittels NMR im Reaktionsgemisch bestimmt. Die nichtflüchtigen Bestandteile des Reaktionsgemisches wurden isoliert und analysiert. Es wurde eine Polymerausbeute von 48 % mit einem Anteil von 14 wt.-% PHB im polymeren Produkt bestimmt.

### Beispiel 14: Carbonylierung von Propylenoxid unter Einsatz von Molybdän(V)-chlorid zur in-situ Generierung eines Katalysator-System

Der Versuch wurde wie in der allgemeinen Versuchsvorschrift beschrieben durchgeführt. 5 mol% Molybdän(V)-chlorid, DME, Naphthalin als interner Standard (1 mmol) und PO (10 mmol, 1,8 M) wurden eingesetzt. Die Reaktion wurde bei 150 °C, 130 bar Anfangsdruck und 68 h Reaktionszeit durchgeführt. Ein PO-Umsatz von 100 % wurde mittels NMR im Reaktionsgemisch bestimmt. Die nichtflüchtigen Bestandteile des Reaktionsgemisches wurden isoliert und analysiert. Es wurde eine Polymerausbeute von 46 % mit einem Anteil von 20 wt.-% PHB im polymeren Produkt bestimmt.

### Beispiel 15: Carbonylierung von Propylenoxid unter Einsatz von Molybdänhexacarbonyl und Salphen-Komplex Salz A zur in-situ Generierung eines Katalysator-System

Der Versuch wurde wie in der allgemeinen Versuchsvorschrift beschrieben durchgeführt. Je 1 mol% er beiden Luft-stabilen Verbindungen Molybdänhexacarbonyl und Salphen-Komplex Salz A, DME, Naphthalin als interner Standard (0,4 mmol) und PO (4 mmol, 1,8 M) wurden eingesetzt. Die Reaktion wurde bei 150 °C, 93 bar Anfangsdruck und 19 h Reaktionszeit durchgeführt. Ein PO-Umsatz von 30 % wurde mittels NMR im Reaktionsgemisch bestimmt. Die nichtflüchtigen Bestandteile des Reaktionsgemisches wurden isoliert und analysiert. Es wurde ein Anteil von 4 wt.-% PHB im polymeren Produkt bestimmt.

### Beispiel 16: Carbonylierung von Propylenoxid unter Einsatz von Katalysator-System 4

Der Versuch wurde wie in der allgemeinen Versuchsvorschrift beschrieben durchgeführt. 3 mol% des Katalysator-System 4, ein 1:7-Gemisch (v/v) aus THF und 1,4-Dioxane als Suspensionsmittel, Naphthalin als interner Standard (0,2 mmol) und PO (2 mmol, 0,5 M) wurden eingesetzt. Die Reaktion wurde bei 150 °C, 124 bar Anfangsdruck und 63 h Reaktionszeit durchgeführt. Ein PO-Umsatz von 73 % und ein Anteil von 8 % Aceton wurde mittels NMR im Reaktionsgemisch bestimmt. Die nichtflüchtigen Bestandteile des Reaktionsgemisches wurden isoliert und analysiert. Es wurde eine Polymerausbeute von 61 % mit einem Anteil von 70 wt.-% PHB im polymeren Produkt bestimmt. Die Massenbilanz bezogen auf PO betrugt 96 %.

### Beispiel 17: Carbonylierung von Propylenoxid unter Einsatz von Katalysator-System 5

Der Versuch wurde wie in der allgemeinen Versuchsvorschrift beschrieben durchgeführt. 1 mol% des Katalysator-System 5, THF, Naphthalin als interner Standard (0,4 mmol) und PO (4 mmol, 0,9 M) wurden eingesetzt. Die Reaktion wurde bei 150 °C, 103 bar Anfangsdruck und 63 h Reaktionszeit durchgeführt. Ein PO-Umsatz von 30 % wurde mittels NMR im Reaktionsgemisch bestimmt. Die nichtflüchtigen Bestandteile des Reaktionsgemisches wurden isoliert und analysiert. Es wurde eine Polymerausbeute von 13 % mit einem Anteil von 25 wt.-% PHB im polymeren Produkt bestimmt.

### Beispiel 18 (Gegenbeispiel): Carbonylierung von Propylenoxid ohne Einsatz eins Katalysator-Systems

Der Versuch wurde wie in der allgemeinen Versuchsvorschrift beschrieben durchgeführt jedoch ohne die Zugabe eines Katalysator-Systems. DME, Naphthalin als interner Standard (1 mmol) und PO (10 mmol, 1,8 M) wurden eingesetzt. Die Reaktion wurde bei 150 °C, 70 bar Anfangsdruck und 5 h Reaktionszeit durchgeführt. Ein PO-Umsatz von 2 % wurde mittels NMR im Reaktionsgemisch bestimmt. Es konnte nur Spuren von Polymer gefunden werden, die jedoch kein PHB enthielten.

### Beispiel 19: Carbonylierung von Propylenoxid unter Einsatz von Molybdänhexacarbonyl

Der Versuch wurde wie in der allgemeinen Versuchsvorschrift beschrieben durchgeführt. 5 mol% Molybdänhexacarbonyl, THF, Naphthalin als interner Standard (1 mmol) und PO (10 mmol, 1,7 M) wurden eingesetzt. Die Reaktion wurde bei 80 °C, 62 bar Anfangsdruck und 24 h Reaktionszeit durchgeführt. Ein PO-Umsatz von 11 % wurde mittels NMR im Reaktionsgemisch bestimmt. Es konnte nur Spuren von Polymer gefunden werden, die jedoch kein PHB enthielten.

### Beispiel 20: Carbonylierung von Propylenoxid unter Einsatz von Molybdänhexacarbonyl

Der Versuch wurde wie in der allgemeinen Versuchsvorschrift beschrieben durchgeführt. 5 mol% Molybdänhexacarbonyl, DME, Naphthalin als interner Standard (1 mmol) und PO (10 mmol, 3,7 M) wurden eingesetzt. Die Reaktion wurde bei 150 °C, 70 bar Anfangsdruck und 67 h Reaktionszeit durchgeführt. Ein PO-Umsatz von 50 % wurde mittels NMR im Reaktionsgemisch bestimmt. Es konnte nur Spuren von Polymer gefunden werden, die jedoch kein PHB enthielten.

### Beispiel 21: Carbonylierung von Propylenoxid unter Einsatz von Molybdänhexacarbonyl und Bortrifluorid

Der Versuch wurde wie in der allgemeinen Versuchsvorschrift beschrieben durchgeführt. 5 mol% Molybdänhexacarbonyl, 5 mol% Bortrifluorid als Etherat, THF, Naphthalin als interner Standard (1 mmol) und PO (10 mmol, 1,7 M) wurden eingesetzt. Die Reaktion wurde bei 80 °C, 62 bar Anfangsdruck und 24 h Reaktionszeit durchgeführt. Ein PO-Umsatz von 100 % und die Bildung von 1 % Propanal und 3 % 2-Methyl-2-pentenal wurden mittels NMR im Reaktionsgemisch bestimmt. Die nichtflüchtigen Bestandteile des Reaktionsgemisches wurden isoliert und analysiert. Es wurde eine Polymerausbeute von 84 % mit einem Anteil von 3 wt.-% PHB im polymeren Produkt bestimmt.

### Beispiel 22: Carbonylierung von Propylenoxid unter Einsatz von Katalysator-System 7

Der Versuch wurde wie in der allgemeinen Versuchsvorschrift beschrieben durchgeführt. 5 mol% des Katalysator-Systems 7, THF, Naphthalin als interner Standard (0,1 mmol) und PO (1 mmol, 0,9 M) wurden eingesetzt. Die Reaktion wurde bei 120 °C, 80 bar Anfangsdruck und 46 h Reaktionszeit durchgeführt. Ein PO-Umsatz von 100 % wurde mittels NMR im Reaktionsgemisch bestimmt. Es konnten lediglich Spuren von PHB nachgewiesen werden.

### Beispiel 23: Carbonylierung von Propylenoxid unter Einsatz von Katalysator-System 8 und Bortrifluorid

Der Versuch wurde wie in der allgemeinen Versuchsvorschrift beschrieben durchgeführt. 2 mol% des Katalysator-Systems 8, 2,4 mol% Bortrifluorid als Etherat, DME, Naphthalin als interner Standard (0,2 mmol) und PO (2 mmol, 0,9 M) wurden eingesetzt. Die Reaktion wurde bei 120 °C, 75 bar Anfangsdruck und 45 h Reaktionszeit durchgeführt. Ein PO-Umsatz von 72 % wurde mittels NMR im Reaktionsgemisch bestimmt. Es konnten lediglich Spuren von PHB nachgewiesen werden.

### Beispiel 24 (Vergleich): Carbonylierung von Propylenoxid unter Einsatz von Katalysator-System 10

Der Versuch wurde wie in der allgemeinen Versuchsvorschrift beschrieben durchgeführt. 1 mol% des Katalysator-Systems 10, THF, Naphthalin als interner Standard (0,2 mmol) und PO (2 mmol, 0,9 M) wurden eingesetzt. Die Reaktion wurde bei 120 °C, 82 bar Anfangsdruck und 15 h Reaktionszeit durchgeführt. Ein PO-Umsatz von 100 %, die Bildung von 77 % Methylbernsteinsäureanhydrid und 2 % Aceton konnte mittels NMR im Reaktionsgemisch nachgewiesen werden. Es konnten lediglich Spuren von Polymer und kein PHB nachgewiesen werden.

Die Charakterisierung und Quantifizierung von Methylbernsteinsäureanhydrid erfolgt anhand der spezifischen ¹H-NMR-Signale und deren Integralen im Verhältnis zum eingesetzten PO/internen Standard:
¹H NMR (CDCl₃, 7.26 ppm): 1.42 (d, CH₃), 2.57-2.67 (m, CH), 3.11-3.23 (m, CH₂) ;
¹³C NMR (CDCl₃, 77.16 ppm): 16.1, 35.7, 36.1, 170.1, 174.6

**Tabelle 1: Ausführungsbeispiele zur Carbonylierung von Epoxiden**

| Bsp.-Nr. | Katalysator-System ([mol%]) | Epoxid | Suspensions-mittel | T [°C] | p [bar] | t [h] | Umsatz Epoxid [%] | Ausbeute Polymer [%] | Anteil PHB in Polymer [wt.-%] |
|---|---|---|---|---|---|---|---|---|---|
| 1 | 1 (1) | PO | THF | 120 | 80 | 23 | 85^{a} | n.b. | n.b. |
| 2 | 1 (1) | PO | THF | 120 | 130 | 48 | n.b. | 14 | 17 |
| 3 | 1 (2) | PO | THF | 100 | 60 | 46 | 65 | n.b. | 17 |
| 4 | 1 (2) | PO | THF | 150 | 100 | 23 | 94 | 50 | 45 |
| 5 | 1 (1) | EO | THF | 150 | 100 | 87 | 98 | 99 | 3^{b} |
| 6 | 1 (1) | PO | DME | 120 | 80 | 19 | n.b. | n.b. | 39 |
| 7 | 1 (2) | PO | DME | 120 | 80 | 19 | 69 | 7 | 14 |
| 8 | 1 (0,1) | PO | DME | 150 | 70 | 65 | 41 | 8 | 58 |
| 9 | 1 (1) | PO | DME | 150 | 70 | 65 | 67 | 45 | 28 |
| 10 | 1 (1) | EO | THF/DME (2:1 v/v) | 120 | 70 | 65 | 97 | 99 | 2^{b} |
| 11 | 1 (0,4) | PO | - | 120 | 80 | 19 | 89 | 8 | 4 |
| 12 | 2 (1) | PO | DME | 150 | 70 | 65 | 54 | 8 | 16 |
| 13 | 3 (1) | PO | THF | 150 | 75 | 21 | 78 | 48 | 14 |
| 14 | Molybdän(V)-chlorid^{c} (5) | PO | DME | 150 | 130 | 68 | 100 | 46 | 20 |
| 15 | Molybdänhexacarbonyl (1) und Salphen-Komplex Salz A^{c} (1) | PO | DME | 150 | 93 | 19 | 30 | n.b. | 4 |
| 16 | 4 (3) | PO | THF/1,4-Dioxane (1:7 v/v) | 150 | 124 | 63 | 73 | 61 | 70 |
| 17 | 5 (1) | PO | THF | 150 | 103 | 63 | 30 | 13 | 25 |
| 18 | - | PO | DME | 150 | 70 | 5 | 2 | Spuren | 0 |
| 19 | Molybdänhexacarbonyl (5) | PO | THF | 80 | 62 | 24 | 11 | Spuren | 0 |
| 20 | Molybdänhexacarbonyl (5) | PO | DME | 150 | 70 | 67 | 50 | Spuren | 0 |
| 21 | Molybdänhexacarbonyl (5) und Bortrifluorid (5) | PO | THF | 80 | 62 | 24 | 100 | 84 | 3 |
| 22 | 7 (5) | PO | THF | 120 | 80 | 46 | 100 | n.b. | Spuren |
| 23 | 8 (2) und Bortrifluorid (2,4) | PO | DME | 120 | 75 | 45 | 72 | n.b. | Spuren |
| 24 (Vgl.) | 10 (1) | PO | THF | 120 | 82 | 15 | 100^{d} | Spuren | 0 |

| | | | | | | | | | |
|---|---|---|---|---|---|---|---|---|---|
| n.b. = nicht bestimmt ^{a}Mittels GC-Analyse des Reaktionsgemischs wurde die Bildung von 3 % *beta*-Butyrolacton nachgewiesen. ^{b}Angabe für den Anteil an PHP im Polymer in wt.-%. ^{c}Precursor zur *in situ*-Bildung eines aktiven Katalysator-Systems. ^{d}Mittels ¹H- und ¹³C-NMR-Analyse des Reaktionsgemischs konnte die Bildung von Methylbernsteinsäureanhydrid mit einer Ausbeute von 77 % nachgewiesen werden. | | | | | | | | | |

## Patentansprüche

1. Verfahren zur Carbonylierung von Epoxiden in Gegenwart von Katalysator-Systemen, **dadurch gekennzeichnet, dass** das Katalysatorsystem eine Molybdän-basierte Verbindung enthält.

2. Verfahren gemäß Anspruch 1, wobei die Carbonylierung in Gegenwart von Kohlenmonoxid erfolgt.

3. Verfahren gemäß Anspruch 1 oder 2, wobei die Molybdän-basierte Verbindung ein oder mehrere Carbonyl-Liganden bevorzugt ein bis sechs Carbonyl-Liganden, besonders bevorzugt zwei bis fünf Carbonyl-Liganden enthält.

4. Verfahren gemäß einem der Ansprüche 1 bis 3, wobei die Molybdän-basierte Verbindung einen weiteren Liganden (L) ungleich des Carbonyl-Liganden aufweist.

5. Verfahren gemäß Anspruch 4, wobei der Ligand (L) eine oder mehrere Verbindung(en) ist und ausgewählt wird aus der Gruppe bestehend aus H, F, Cl, Br, I, CN, NC, SCN, N₃, NO₂, NO₃, NH₂, OTf, OAc, OH, HSO₄, η³-C₃H₅, Butadiene (C₄C₆), Cyclopentadienyl (Cp, η⁵-C₅H₅), Pentamethylcyclopentadienyl (Cp*, η³-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), NH₃, Ethylendiamin, Diethylentriamin, Tetramethylethylendiamin, Anilin, Pyridin, 2,2'-Bipyridin, PPh₃, PMe₃, PEt₃, PBu₃, PH₃, P(OMe)₃, P(OEt)₃, Diethylether, THF und 2-Me-THF, bevorzugt H, F, Cl, Br, I, CN, NC, SCN, N₃, NO₂, NO₃, NH₂, OTf, OAc, η³-C₃H₅, Butadiene (C₄C₆), Cyclopentadienyl (Cp, η⁵-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben), 1,3-Bis(2,4,6-trimethylphenyl)-4,5-dihydroimidazol-2-ylidene (SIMes, NHC-Carben), Ethylendiamin, Diethylentriamin, Tetramethylethylendiamin, Anilin, Pyridin, 2,2'-Bipyridin, PPh₃ und PMe₃, besonders bevorzugt H, F, Cl, Br, I, CN, NC, SCN, N₃, OTf, Cyclopentadienyl (Cp, η⁵-C₅H₅), Pentamethylcyclopentadienyl (Cp*, η⁵-C₅Me₅), C(Ph)(Ph) (als Fischer-Carben), C(OMe)(Ph) (als Fischer-Carben), C(OEt)(NHPh) (als Fischer-Carben), 1,3-Dimesitylimidazol-2-ylidene (IMes, NHC-Carben) und 1,3-Bis(2,4,6-trimethylphenyl)-4,5-dihydroimidazol-2-ylidene (SIMes, NHC-Carben), ganz besonders bevorzugt Cl, Br und Pentamethylcyclopentadienyl (Cp*, η⁵-C₅Me₅).

6. Verfahren gemäß einem der Ansprüche 1 bis 5, wobei das Molybdän in der Molybdän-basierten Verbindung eine Oxidationsstufe von null aufweist.

7. Verfahren gemäß einem der Ansprüche 1 bis 6 wobei das Katalysator-System eine zusätzliche Lewis Säure enthält.

8. Verfahren gemäß Anspruch 7, wobei die Lewis-Säure kationisch oder ladungsneutral, bevorzugt kationisch ist.

9. Verfahren gemäß Anspruch 8, wobei die Lewis-Säure kationisch ist

10. Verfahren gemäß Anspruch 9, wobei die kationische Lewis-Säure ein unsubstituiertes Dicyclopentadienyl-Metall-Kation, ein substituiertes Dicyclopentadienyl-Metall-Kation, ein unsubstituiertes Metall-Porphyrin-Kation, ein substituiertes Metall-Porphyrin-Kation, ein unsubstituiertes Metall-Salen-Kation, ein substituiertes Metall-Salen-Kation, ein unsubstituiertes Metall-Salphen-Kation und/oder ein und substituiertes Metall-Salphen-Kation ist.

11. Verfahren gemäß Anspruch 9 oder 10, wobei die Molybdän-basierte Verbindung anionisch ist.

12. Verfahren gemäß einem der Ansprüche 1 bis 11, wobei das Katalysatorsystem die Struktur (I), (II), (III), (IV), (V), (VI), und/oder (VII) aufweist: 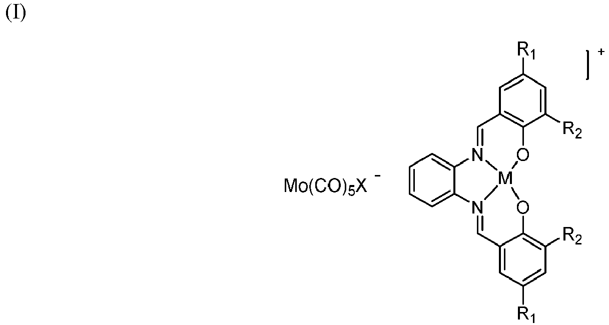
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl; 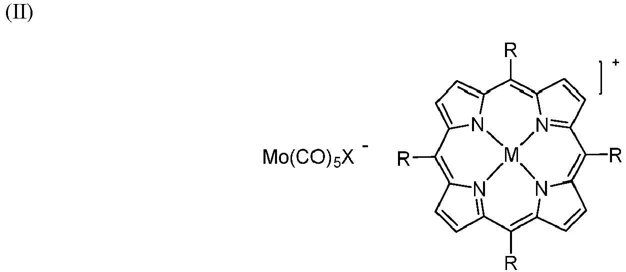
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R ausgewählt aus der Gruppe von Wasserstoff (-H), Methyl, tert. Butyl, Methoxy, Phenyl, 4-Methylphenyl, 4-Methoxyphenyl, 4-Chlorphenyl, 4-Bromphenyl, 4-Carboxylphenyl, 3,5-Dimethoxyphenyl, 2-Pyridyl, 4-Pyridyl und N-Methyl-4-pyridyl, bevorzugt tert. Butyl, Methoxy, Phenyl, 4-Methylphenyl, 4-Methoxyphenyl, 4-Chlorphenyl oder 3,5-Dimethoxyphenyl, ganz besonders bevorzugt Phenyl. 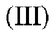 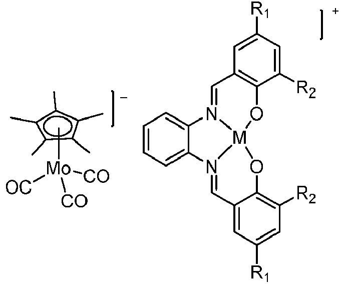
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl; 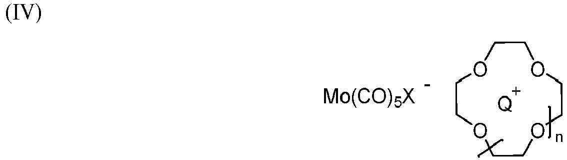
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
mit Q = Li, Na, K, Rb, Cs, Cu oder Ag, bevorzugt Li, Na, K oder Rb, ganz besonders bevorzugt K;
mit n = 1-5, bevorzugt 2-5, ganz besonders bevorzugt 3; 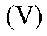 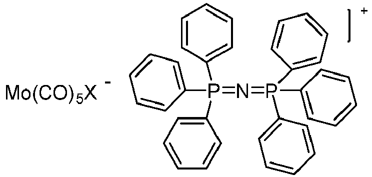
mit X = H, F, Cl, Br, I, CN, NC, SCN, NCS, CP, N₃, NO₂, NO₃, NH₂, OTf, OH oder HSO₄, bevorzugt H, F, Cl, Br, I, CN, N₃, oder OTf, ganz besonders bevorzugt Cl oder Br;
(VI) Q[Mo(CO)₅]
mit Q = Li, Na, K, Rb, Cs, Cu oder Ag, bevorzugt Li, Na, K oder Rb, ganz besonders bevorzugt Na;
und /oder (VII) 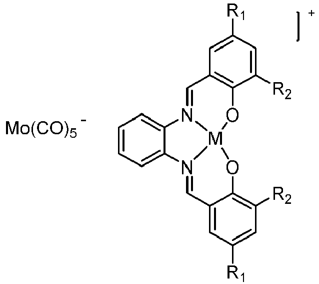
mit M = Cr(III), Al(III), Fe(III), Co(III), Mn(III), V(III), In(III), Ga(III), Y(III), Ru(III), La(III), Ce(III), Gd(III) oder Ir(III), bevorzugt Cr(III), Al(III), Fe(III), Co(III), Mn(III), oder Ga(III), ganz besonders bevorzugt Cr(III) oder Al(III);
mit R₁ und R₂ unabhängig voneinander ausgewählt aus der Gruppe von Wasserstoff (-H), Methyl, tert. Butyl, Phenyl, Nitro, Brom, Chlor, Hydroxy, Diethylamino und Methoxy bevorzugt R₁ gleich R₂ ausgewählt aus der Gruppe von Wasserstoff (-H), Methyl oder tert. Butyl, ganz besonders bevorzugt tert. Butyl.

13. Carbonylierungsprodukte erhältlich nach mindestens einem der Ansprüche 1 bis 12, wobei der molare Anteil an cyclischen Anhydriden bezogen auf das eingesetzte Epoxid weniger als 5 mol% beträgt, wobei der Anteil an cyclischen mittels der im Experimentalteil offenbarten ¹H-NMR Methode bestimmt wurde.

14. Verfahren zur Herstellung von Carbonylierungsfolgeprodukten, bevorzugt Polyurethanen, durch Umsetzung der Carbonylierungsprodukte gemäß Anspruch 13 mit Epoxiden, Polyisocyananten und/oder Polycarbonsäuren, bevorzugt mit Polyisocyanaten.

15. Verwendung der Katalysator-Systeme enthaltend eine Molybdän-basierte Verbindung gemäß einem der Ansprüche 1 bis 12 zur Carbonylierung von Epoxiden.
